# EUROPEAN PATENT SPECIFICATION

(11) **EP 2 802 290 B1**
(45) Date of publication and mention of the grant of the patent: **11.07.2018**
(21) Application number: 13736314.9
(22) Date of filing: 10.01.2013
(51) Int. Cl.: A61F 2/24

(54) **ARTICULATED SUPPORT STRUCTURE WITH SECONDARY STRUT FEATURES**
GELENKIGE TRAGESTRUKTUR MIT SEKUNDÄRER STREBE
STRUCTURE DE SUPPORT ARTICULÉE DOTÉE D'ÉLÉMENTS DE RENFORCEMENT SECONDAIRES

(30) Priority: 10.01.2012 US 201261585165 P
(43) Date of publication of application: 19.11.2014
(60) Divisional application: 17201394.8
(73) Proprietor: Jenesis Surgical, LLC, Charlestown, RI 02813 (US)
(72) Inventor: White, Jennifer K., Charlestown, RI 02813 (US)
(74) Representative: J A Kemp
(86) International application number: PCT/US2013/021052
(87) International publication number: WO 2013/106585

(56) References cited:
- WO-A1-2013/059776
- WO-A2-2008/015257
- US-A1- 2008 004 696
- US-A1- 2009 132 035
- US-A1- 2011 230 956
- US-A1- 2011 288 629
- US-B2- 6 530 952
- US-B2- 7 097 658

## Description

### BACKGROUND

Endoluminal stents can be implanted in a vessel or tract of a patient to help maintain an open lumen. The stents can also be used as a frame to support a prosthetic device or to deliver a therapeutic agent. Stents can be implanted by either an open operative procedure or a closed operative procedure. When an option exists, the less invasive closed procedure is generally preferred because the stent can be guided through a body lumen, such as the femoral artery, to its desired location.

Closed procedures typically use one of two techniques. One closed procedure employs balloon catheterization where an expandable stent encloses an inflatable balloon. In this procedure, the stent is implanted by inflating the balloon, which causes the stent to expand. The actual positioning of the stent cannot be determined until after the balloon is deflated and, if there is a misplacement of the stent, the process cannot be reversed to reposition the stent.

The other closed procedure employs a compressed stent enclosed by a removable sheath. In this procedure, a stent made from a shape memory alloy, such as Nitinol, is held in a compressed state by a sheath. The stent is implanted by withdrawing the sheath, causing the stent to expand to its nominal shape. Again, if there is a misplacement of the stent, the process cannot be reversed to reposition the stent.

Positioning errors are particularly dangerous when the stent is used to support a cardiac valve. Serious complications and patient deaths have occurred due to malpositioning of the valve at the implant site in the body, using the available stent-mounted valves. Malpositioning of the valve has resulted in massive paravalvular leakage, device migration, and coronary artery obstruction. The majority of these complications were unavoidable, but detected at the time of the procedure. However, due to inability to reposition or retrieve the device, these problems were impossible to reverse or mitigate during the procedure.

US 2011/0230956 A1 discloses a support structure in accordance with the precharacterizing section of claim 1.

### SUMMARY

According to an aspect of the present invention there is provided the biocompatible articulated support structure of claim 1.

Additional aspects of the invention are set out in the dependent claims. Insofar as the term "invention" and or "embodiment" are used in the following, and/or features are presented as being optional, this should be interpreted in such a way that the only protection sought is that of the invention as claimed.

An endoluminal support structure or stent in accordance with certain embodiments of the invention solves certain deficiencies found in the prior art. In particular, the support structure can be repositioned within the body lumen or retrieved from the lumen.

A particular embodiment of the invention includes a support apparatus implantable within a biological lumen. The support apparatus can include a plurality of elongated strut members interlinked by a plurality of swivel joints, wherein the swivel joints can cooperate with the stent members to adjustably define a shaped structure between a compressed orientation and an expanded orientation.

More particularly, the shaped structure can be one of a cylindrical, a conical, or an hourglass shape. A swivel joint can form a scissor mechanism with a first strut member and a second strut member. Furthermore, the strut members can be arranged as a series of linked scissor mechanisms. The apparatus can further include an actuation mechanism to urge the swivel joints within a range of motion.

The apparatus can also include a prosthetic valve coupled to the shaped structure.

Another particular embodiment of the invention can include a medical stent implantable within a biological lumen. The medical stent can include a plurality of elongated strut members, including a first strut member and a second strut member, and a swivel joint connecting the first strut member and the second strut member.

In particular, the swivel joint can form a scissor mechanism with the first strut member and the second strut member. The swivel joint can bisect the first strut member and the second strut member. The swivel joint can interconnect a first end of the first strut member with a first end of the second strut member.

The plurality of strut members can be arranged as a series of linked scissor mechanisms. The strut members can also be non-linear. The strut members can be arranged to form one of a cylindrical, a conical, or an hourglass shape.

The stent can further include an adjustment mechanism to exerting a force to urge the strut members about the swivel joint within a range of motion.

The stent can include a prosthetic valve coupled to the strut members.

Specific embodiments of the invention can include prosthetic valves that are rotatable or conventional.

A rotatable prosthetic valve can include a first structural member coupled to the strut members, a second structural member rotatable relative to the first structural member, and a plurality of pliable valve members connecting the first structural member with the second structural member such that rotation of the second structural member relative to the first structural member can urge the valve members between an open and a closed state. In particular, the rotation of the second structural member can be responsive to the natural flow of a biological fluid.

A conventional prosthetic valve can include a plurality of pliable valve leaflets having commissures at the intersection of two strut members. The prosthetic valve can further include a skirt material coupled to the strut members.

Another particular embodiment of the invention can include a splint device. The splint device can include a plurality of elongated strut members, including a first strut member and a second strut member, and a pivot joint connecting the first strut member and the second strut member.

In particular, the pivot joint can form a scissor mechanism with the first strut member and the second strut member. The pivot joint can bisect the first strut member and the second strut member. The pivot joint can interconnect a first end of the first strut member with a first end of the second strut member.

The plurality of strut members can be helical and can be arranged to form a cylindrical shape. The cylindrical shape can be connected to a plurality of strut members forming a tapered shape.

The splint device can further include a polymer coating. The polymer coating can cause the device to be self-expanding.

A variation of the splint device can have a constriction element to create constrictive pressure.

Another particular embodiment of the invention can include a ring device. The ring device can include a plurality of elongated strut members, including a first strut member and a second strut member, and a pivot joint connecting the first strut member and the second strut member.

In particular, the pivot joint can form a scissor mechanism with the first strut member and the second strut member. The pivot joint can bisect the first strut member and the second strut member. The pivot joint can interconnect a first end of the first strut member with a first end of the second strut member.

The plurality of strut members can be curved and can be arranged to form a cylindrical shape.

The ring device can further include a polymer coating. The polymer coating may cause the device to be self-expanding.

A variation of the ring device can have a constricting element to create constrictive pressure.

A particular advantage of a support structure in accordance with embodiments of the invention is that it enables a prosthetic valve to be readily retrieved and repositioned in the body. If following deployment, the valve is malpositioned or deemed dysfunctional, the support structure allows the valve to be readily repositioned and re-deployed at a new implant site, or removed from the body entirely. This feature of the device can prevent serious complications and save lives by enabling the repair of mal-positioned devices in the body.

There is herein disclosed a biocompatible articulated structure, comprising a tubular structure, which comprises, a central lumen, a central axis, a plurality of discrete inner struts, a plurality of discrete outer struts, and at least two radial struts, wherein each of the plurality of discrete inner struts and the plurality of discrete outer struts comprises first end, a second end, and a net length therebetween, wherein each of the at least two radial struts comprises an outer end, an inner end and a net length therebetween, wherein each outer end may be coupled to at least one strut selected from the plurality of discrete inner struts and the plurality of discrete outer struts, and wherein the inner ends of the at least two radial struts are coupled together, wherein each of the plurality of discrete inner struts comprises articulations with at least three different discrete outer struts of the plurality of discrete outer struts, wherein each of the plurality of discrete outer struts comprises articulations with at least three different discrete inner struts of the plurality of discrete inner struts, wherein no discrete inner strut of the plurality of discrete inner struts comprises articulations with any other discrete inner strut of the plurality of discrete inner struts, and wherein no discrete outer strut of the plurality of discrete outer struts comprises articulations with any other discrete outer strut of the plurality of discrete outer struts. The inner ends of the at least two radial struts are coupled at centrally aligned coupling apertures. The inner ends of the at least two radial struts may be coupled at coupling apertures using a loop coupling structure. The at least two radial struts may comprise a first plurality of radial struts and a second plurality of radial struts, wherein each outer end of the first plurality of radial struts may be coupled to the first ends of at least one strut selected from the plurality of discrete inner struts and the plurality of discrete outer struts, and wherein each outer end of the second plurality of radial struts may be coupled to the second ends of at least one strut selected from the plurality of discrete inner struts and the plurality of discrete outer struts. The inner ends of the first plurality of radial struts are coupled together and the inner ends of the second plurality of radial struts are coupled together. The inner ends of the first plurality of radial struts are attached to a first deployment structure and the inner ends of the second plurality of radial struts are attached to a second deployment structure. The inner ends of the first plurality of radial struts are attached to a first region of a deployment structure and the inner ends of the second plurality of radial struts are attached to a second region of the deployment structure. The deployment structure may comprise a screw drive mechanism. The biocompatible articulated structure may further comprise a delivery catheter permanently attached to the tubular structure. The delivery catheter may comprise a plurality of wires electrically coupled to the tubular structure.

There is also herein disclosed a biocompatible articulated structure, comprising a tubular structure, which comprising a central lumen, a central axis, a plurality of discrete inner struts, and a plurality of discrete outer struts, wherein each of the plurality of discrete inner struts and the plurality of discrete outer struts comprises first end, a second end, and a net length therebetween, wherein each of the plurality of discrete inner struts comprises articulations with at least four different discrete outer struts of the plurality of discrete outer struts, wherein each of the plurality of discrete outer struts comprises articulations with at least four different discrete inner struts of the plurality of discrete inner struts, wherein no discrete inner strut of the plurality of discrete inner struts comprises articulations with any other discrete inner strut of the plurality of discrete inner struts, wherein no discrete outer strut of the plurality of discrete outer struts comprises articulations with any other discrete outer strut of the plurality of discrete outer struts, wherein at least one strut from either the plurality of discrete inner struts or the plurality of discrete outer struts comprises first end, a second end, and a net length therebetween, and wherein the plurality of discrete inner struts, the plurality of discrete outer struts and the articulations therebetween intrinsically provide a self-expansion force. The tubular structure may comprise an intrinsically stable non-expanding collapsed state. The plurality of discrete inner struts and the plurality of discrete outer struts may be configured to form a first set of cells aligned along a first perimeter of the tubular structure and a second set of cells directly adjacent to the first set of cells and aligned along a second perimeter of the tubular structure. Each of the plurality of discrete inner struts may comprise articulations with at least five different discrete outer struts of the plurality of discrete outer struts, wherein each of the plurality of discrete outer struts comprises articulations with at least five different discrete inner struts of the plurality of discrete inner struts, and wherein the plurality of discrete inner struts and the plurality of discrete outer struts are further configured to form a third set of cells directly adjacent to the second set of cells and aligned along a third perimeter of the tubular structure.

There is furthermore herein disclosed an appendage splint is provide, comprising a frame with an articulable structure comprising a central lumen, a plurality of discrete inner struts, a plurality of discrete outer struts, wherein each of the plurality of discrete inner struts comprises articulations with at least three different discrete outer struts of the plurality of discrete outer struts, wherein each of the plurality of discrete outer struts comprises articulations with at least three different discrete inner struts of the plurality of discrete inner struts, wherein no discrete inner strut of the plurality of discrete inner struts comprises articulations with any other discrete inner strut of the plurality of discrete inner struts, and wherein no discrete outer strut of the plurality of discrete outer struts comprises articulations with any other discrete outer strut of the plurality of discrete outer struts. The frame with an articulable structure may further comprise a cylindrical region and a tapered region. The frame with an articulable structure may further comprise a plurality of discrete curved struts, wherein each of the plurality of curved struts comprises articulations with each other curved strut in the plurality of curved struts, and wherein each of the plurality of curved struts comprises articulations with at least one of the plurality of discrete inner struts and at least one of the plurality of discrete outer struts.

### BRIEF DESCRIPTION OF THE DRAWINGS

The foregoing and other objects, features and advantages of the invention will be apparent from the following more particular description of particular embodiments of the invention, as illustrated in the accompanying drawings in which like reference characters refer to the same parts throughout the different views. The drawings are not necessarily to scale, emphasis instead being placed upon illustrating the principles of the invention.
FIG. 1 is a perspective view of a particular endoluminal support structure.
FIG. 2 is a perspective view of a four strut section of the stent of FIG. 1.
FIG. 3 is a perspective view of a compressed support structure of FIG. 1.
FIG. 4 is a perspective view of the support structure of FIG. 1 in a fully expanded state.
FIG. 5 is a perspective view of the support structure of FIG. 2 having a particular actuator mechanism.
FIG. 6 is a perspective view of the support structure of FIG. 2 having another particular actuator mechanism.
FIG. 7 is a perspective view of a particular support structure and control catheter assembly usable with the actuator mechanisms of FIGS. 5 and 6.
FIG. 8 is a perspective view of a particular rotating prosthetic valve assembly.
FIG. 9 is a perspective view of the valve assembly of FIG. 8 while being closed.
FIG. 10 is a perspective view of the valve assembly of FIG. 8 once completely closed.
FIG. 11 is a perspective view of the valve of FIGS. 8-10 in combination with the support structure of FIG. 1.
FIG. 12 is a perspective view of the valve of FIG. 11 in the open position.
FIG. 13 is a perspective view of a traditional tissue valve mounted to the support structure of FIG. 1.
FIG. 14 is a perspective view of the valve structure of FIG. 13 having a full inner skirt.
FIG. 15 is a perspective view of the valve structure of FIG. 13 having a full outer skirt.
FIG. 16 is a perspective view of the arrangement of strut members in a conical-shaped support structure configuration.
FIG. 17 is a perspective view of an hourglass-shaped support structure configuration.
FIGS. 18A and 18B are perspective and side elevational views, respectively, of one embodiment of an articulated support structure comprising strut extension segments.
FIGS. 19A and 19B are perspective and side elevational views, respectively, of one embodiment of an articulated support structure comprising inner and outer bow struts.
FIG. 20 depicts a perspective view of an embodiment of a self-expanding articulated structure comprising a multi-level configuration.
FIG. 21 depicts a perspective view of an embodiment of an articulated support structure comprising centrally attached radial struts.
FIGS. 22A and 22B are schematic superior views of alternate embodiments of interconnection configurations of the centrally attached radial struts in FIG. 21.
FIG. 23 is a perspective view of an embodiment of an articulated support structure comprising dual deployment wires.
FIG. 24 a perspective view of an embodiment of an articulated support structure comprising a deployment shaft.
FIG. 25A is a side view of an embodiment of an articulated support structure comprising a cylindrical proximal region and a closed tapered distal region and a latex coating.
FIG. 25B is a side view of an embodiment of an articulated support structure comprising a cylindrical proximal region and a closed tapered distal region and a latex coating, shown with a condom.
FIG. 25C is a perspective view of an embodiment of an articulated support structure comprising a cylindrical proximal region and a closed tapered distal region and a latex coating, shown with a condom or sheath.
FIG. 26 is a perspective view of an embodiment of an articulated support structure with a polymer coating.

### DETAILED DESCRIPTION

Particular embodiments of the invention include endoluminal support structures (stents) and prosthetic valves.

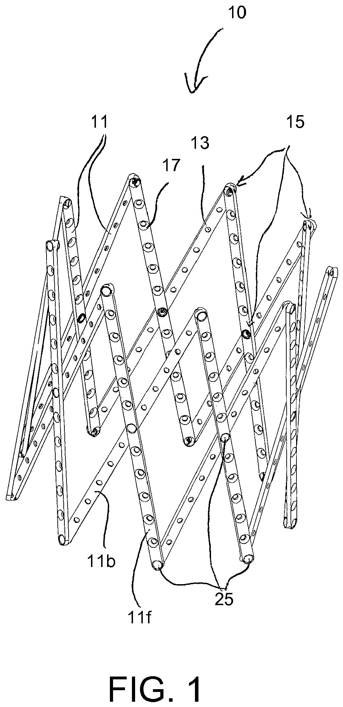

FIG. 1 is a perspective view of a particular endoluminal support structure. As shown, the support structure 10 is a medical stent that includes a plurality of longitudinal strut members 11 interconnected by a plurality of swivel joints 15. In particular, the swivel joints 15 allow the interconnected strut members 11 to rotate relative to each other. As shown, there are eighteen struts 11.

The strut members 11 are fabricated from a rigid or semi-rigid biocompatible material, such as plastics or other polymers and metal alloys, including stainless steel, tantalum, titanium, nickel-titanium (e.g. Nitinol), and cobalt-chromium (e.g. ELGILOY). The dimensions of each strut can be chosen in accordance with its desired use. In a particular embodiment, each strut member is made from stainless steel, which is 0.005-0.020 inch thick. More particularly, each strut is 0.010 inch thick 300 series stainless steel. While all struts 11 are shown as being of uniform thickness, the thickness of a strut can vary across a strut, such as a gradual increase or decrease in thickness along the length of a strut. Furthermore, individual struts can differ in thickness from other individual struts in the same support structure.

As shown, each strut member 11 is bar shaped and has a front surface 11f and a back surface 11b. The strut members can however be of different geometries. For example, instead of a uniform width, the struts can vary in width along its length. Furthermore, an individual strut can have a different width than another strut in the same support structure. Similarly, the strut lengths can vary from strut to strut within the same support structure. The particular dimensions can be chosen based on the implant site.

Furthermore, the struts can be non-flat structures. In particular, the struts can include a curvature, such as in a concave or convex manner in relationship to the inner diameter of the stent structure. The struts can also be twisted. The nonflatness or flatness of the struts can be a property of the material from which they are constructed. For example, the struts can exhibit shape-memory or heat- responsive changes in shape to the struts during various states. Such states can be defined by the stent in the compressed or expanded configuration.

Furthermore, the strut members 11 can have a smooth or rough surface texture. In particular, a pitted surface can provide tensile strength to the struts. In addition, roughness or pitting can provide additional friction to help secure the support structure at the implant site and encourage irregular encapsulation of the support structure 10 by tissue growth to further stabilize the support structure 10 at the implant site over time.

In certain instances, the stent could be comprised of struts that are multiple members stacked upon one another. Within the same stent, some struts could include elongated members stacked upon one another in a multi-ply configuration, and other struts could be one-ply, composed of single-thickness members. Within a single strut, there can be areas of one-ply and multi-ply layering of the members.

Each strut member 11 also includes a plurality of orifices 13 spaced along the length of the strut member 11. On the front surface 11f, the orifices are countersunk 17 to receive the head of a fastener. In a particular embodiment, there are thirteen equally spaced orifices 13 along the length of each strut member 11, but more or less orifices can be used. The orifices 13 are shown as being of uniform diameter and uniform spacing along the strut member 11, but neither is required.

The strut members 11 are arranged as a chain of four-bar linkages. The strut members 11 are interconnected by swivelable or pivot fasteners 25, such as rivets, extending through aligned orifices 13. It should be understood that other swivelable or pivot fasteners 25 can be employed such as screws, bolts, ball-in-socket structures, nails, or eyelets, and that the fasteners can be integrally formed in the struts 11 such as a peened semi-sphere interacting with an indentation or orifice, or a male-female coupling. In addition to receiving a fastener, the orifices 13 also provide an additional pathway for tissue growth-over to stabilize and encapsulate the support structure 10 over time.

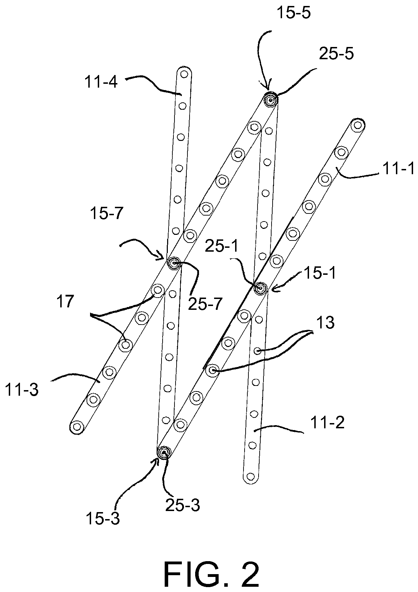

FIG. 2 is a perspective view of a four strut section of the stent of FIG. 1. As shown, two outer strut members 11-1, 11-3 overlap two inner strut members 11-2, 11-4, with their back surfaces in communication with each other.

In particular, the first strut member 11-1 is swivelably connected to the second strut member 11-1 by a middle swivel joint 15-1 using a rivet 25-1, which utilizes orifices 13 that bisect the strut members 11-1, 11-2. Similarly, the third strut member 11-3 is swivelably connected to bisect the fourth strut member 11-4 by a middle swivel joint 15-7 using a rivet 25-7. It should be understood that the middle swivel joints 15-1, 15-7 function as a scissor joint in a scissor linkage or mechanism. As shown, the resulting scissor arms are of equal length. It should also be understood that the middle joint 15-1, 15-7 need not bisect the joined strut members, but can instead utilize orifices 13 offset from the longitudinal centers of the strut members resulting in unequal scissor arm lengths.

In addition to the middle scissor joint 15-1, the first strut member 11-1 is swivelably connected to the third strut member 11-3 by a distal anchor swivel joint 15-5, located near the distal ends of the strut members 11-1, 11-3. Similarly, the first strut member 11-1 is swivelably connected to the fourth strut member 11-4 by a proximal anchor swivel joint 15-3, located near the proximal ends of the strut members 11-1, 11-4. To reduce stresses on the anchor rivets 25-3, 25-5, the distal and proximal ends of the struts 11 can be curved or twisted to provide a flush interface between the joined struts.

As can be seen, the support structure 10 (FIG. 1) is fabricated by linking together a serial chain of scissor mechanisms. The chain is then wrapped to join the last scissor mechanism with the first scissor mechanism in the chain. By actuating the linkage the links can be opened or closed, which results in expanding or compressing the stent 10 (FIG. 1).

Returning to FIG. 1, by utilizing the swivel joints 15, the diameter of the stent can be compressed for insertion through a biological lumen, such as an artery, to a selected position. The stent can then be expanded to secure the stent at the selected location within the lumen. Furthermore, after being expanded, the stent can be recompressed for removal from the body or for repositioning within the lumen.

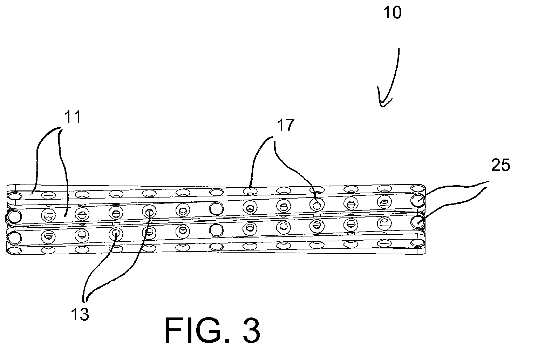

FIG. 3 is a perspective view of a compressed support structure of FIG. 1. When compressed, the stent 10 is at its maximum length and minimum diameter. The maximum length is limited by the length of the strut members, which in a particular embodiment is 15 mm. The minimum diameter is limited by the width of the strut members, which in a particular embodiment is 0.052 inch.

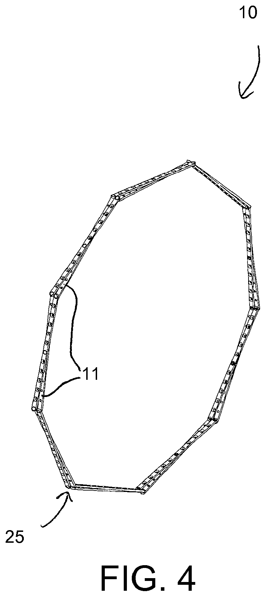

FIG. 4 is a perspective view of the support structure of FIG. 1 in a fully expanded state. As shown, the fully expanded support structure 10 forms a ring, which can be used as an annuloplasty ring. In particular, if one end of the stent circumference is attached to tissue, the compression of the stent will enable the tissue to cinch. Because the stent has the ability to have an incremental and reversible compression or expansion, the device could be used to provide an individualized cinching of the tissue to increase the competency of a heart valve. This could be a useful treatment for mitral valve diseases, such as mitral regurgitation or mitral valve prolapse.

While the support structure 10 can be implanted in a patient during an open operative procedure, a closed procedure will often be more desirable. As such, the support structure 10 can include an actuation mechanism to allow a surgeon to expand or compress the support structure from a location remote from the implant site. Due to the properties of a scissor linkage wrapped into a cylinder (FIG. 1), actuation mechanisms can exert work to expand the stent diameter by either increasing the distance between neighboring scissor joints, and decreasing the distance between the anchor joints.

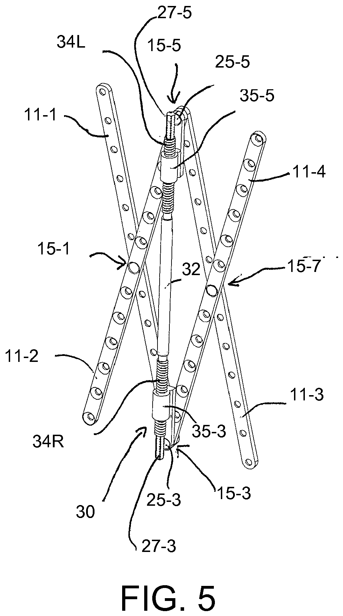

FIG. 5 is a perspective view of the support structure of FIG. 2 having a particular actuator mechanism. As shown, the actuator mechanism 30 includes a dual-threaded rod 32 positioned on the inside of the support structure 10 (FIG. 1). It should be understood, however, that the actuator mechanism 30 can instead be positioned on the outside of the support structure 10. Whether positioned on the inside or outside, the actuator mechanism 30 operates in the same way. The rod includes right-hand threads 34R on its proximal end and left-hand threads 34L on its distal end. The rod 32 is mounted the anchor points 15-3, 15-5 using a pair of threaded low-profile support mounts 35-3, 35-5. Each end of the rod 32 is terminated by a hex head 37-3, 37-5 for receiving a hex driver (not shown). As should be understood, rotating the rod 32 in one direction will urge the anchor points 25-3, 25-5 outwardly to compress the linkages while rotating the rod 32 in the opposite direction will urge the anchor points 25-3, 25-5 inwardly to expand the linkages.

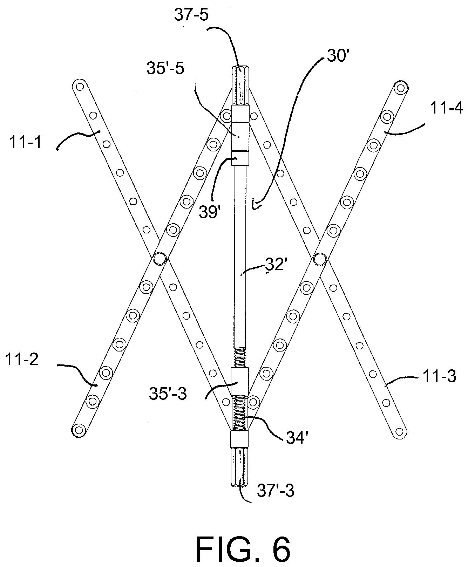

FIG. 6 is a perspective view of the support structure of FIG. 2 having another particular actuator mechanism. As shown, the actuator mechanism 30' includes a single-threaded rod 32' positioned on the inside of the support structure 10 (FIG. 1). The rod 32' includes threads 34' on one of its ends. The rod 32' is mounted to low profile anchor points 15-3, 15-5 using a pair of support mounts 35'-3, 35'-5, one of which is threaded to mate with the rod threads 34'. The unthreaded end of the rod 32' includes a retaining stop 39' that bears against the support mount 35'-5 to compress the support structure. Each end of the rod 32' is terminated by a hex head 37'-3, 37'-5 for receiving a hex driver (not shown). Again, rotating the rod 32' in one direction will urge the anchor points 25-3, 25-5 outwardly to compress the linkages while rotating the rod 32' in the opposite direction will urge the anchor points 25-3, 25-5 inwardly to expand the linkages.

In addition, because the struts overlap, a ratcheting mechanism can be incorporated to be utilized during the sliding of one strut relative to the other. For example, the stent could lock at incremental diameters due to the interaction of features that are an integral part of each strut. An example of such features would be a male component (e.g. bumps) on one strut surface which mates with the female component (e.g. holes) on the surface of the neighboring strut surface, as the two struts slide pass one another. Such structures could be fabricated to have an orientation, such that they incrementally lock the stent in the expanded configuration as the stent is expanded. Such a stent could be expanded using a conventional balloon or other actuation mechanism described in this application.

Because the support structure 10 of FIGS. 5 and 6 are intended to be implanted during a closed surgical procedure, the actuator mechanism is controlled remotely by a surgeon. In a typical procedure, the support structure 10 is implanted through a body lumen, such as the femoral artery using a tethered endoluminal catheter. As such, the actuator mechanism 30 can be controlled via the catheter.

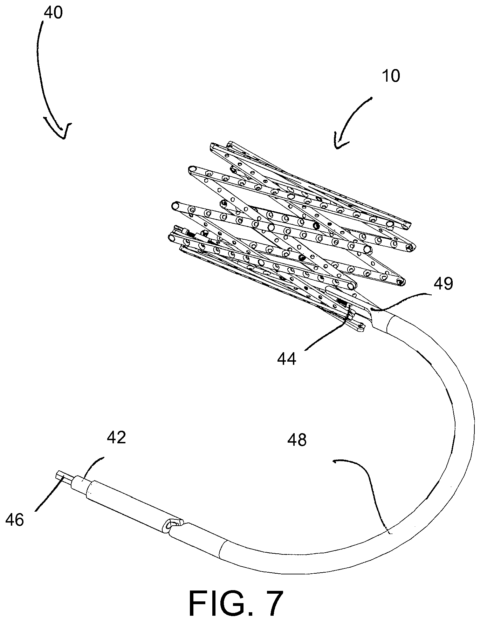

FIG. 7 is a perspective view of a particular support structure and control catheter assembly usable with the actuator mechanisms of FIGS. 5 and 6. The control catheter 40 is dimensioned to be inserted with the support structure through a biological lumen, such as a human artery. As shown, the control catheter 40 includes a flexible drive cable 42 having a driver 44 on its distal end that removably mates with a hex head 37, 37' of the actuator mechanism (FIGS. 5 and 6). The proximal end of the cable 42 includes a hex head 46. In operation, the proximal hex head 46 of the cable 42 is rotated by a surgeon, using a thumb wheel or other suitable manipulator (not shown). Rotation of the hex head 46 is transferred by the cable 42 to the driver head 44 to turn the actuator rod 30, 30' (FIGS. 5 and 6).

The cable 42 is encased by a flexible outer sheath 48. The distal end of the outer sheath 48 includes a lip or protuberance 49 shaped to interface with the support structure 10. When the cable 42 is turned, the outer sheath lip 49 interacts with the support structure 10 to counteract the resulting torque.

By employing threads, the rod is self-locking to maintain the support structure in the desired diameter. In a particular embodiment, the rod 32, 32' has a diameter of 1.0 mm and a thread count of 240 turns/inch. While a threaded rod and drive mechanism are described, other techniques can be employed to actuate the linkages depending on the particular surgical application. For example, the actuator mechanism can be disposed within the thickness of the strut members, instead of inside or outside of the stent. For example, worm gears or a rack and pinion mechanism can be employed as known in the art. One of ordinary skill in the art should recognize other endoluminal actuation techniques. In other situations, the support structure can be implanted during an open procedure, which may not require an external actuation mechanism.

Although there are other uses for the described support structure, such as drug delivery, a particular embodiment supports a prosthetic valve. In particular, the support structure is used in combination with a prosthetic valve, such as for an aortic valve replacement.

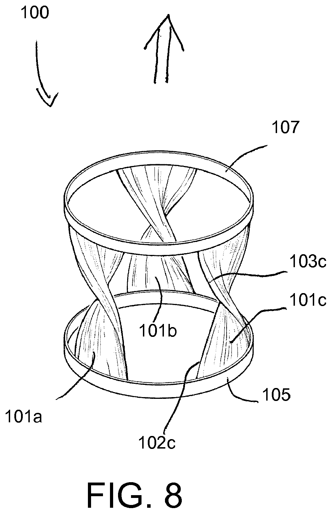

FIG. 8 is a perspective view of a particular rotating prosthetic valve assembly. The prosthetic valve 100 comprises a three leaflet configuration shown in an open position. The leaflets are derived from a biocompatible material, such as animal pericardium (e.g. bovine, porcine, equine), human pericardium, chemically treated pericardium, gluteraldehyde-treated pericardium, tissue engineered materials, a scaffold for tissue engineered materials, autologous pericardium, cadaveric pericardium, Nitinol, polymers, plastics, PTFE, or any other material known in the art.

The leaflets 101a, 101b, 101c are attached to a stationary cylindrical member 105 and a non-stationary cylindrical member 107. One side of each leaflet 101 is attached to the non-stationary cylindrical member 107. The opposing side of each leaflet 101 is attached to the stationary cylindrical member 105. The attachment of each leaflet 101 is in a direction generally perpendicular to the longitudinal axis of the cylindrical members 105, 107. In this embodiment, each leaflet 101 is pliable, generally rectangular in shape, and has a 180 degree twist between its attachments to stationary member 105 and non-stationary member 107. Each leaflet 101 has an inner edge 102 and an outer edge 103, with the edges 102c, 103c of one leaflet 101c being referenced in the figure. As known in the art, the leaflets can be fabricated from either biological or non-biological materials, or a combination of both.

One way to actuate the valve to close is by utilizing the forces exerted by the normal blood flow or pressure changes of the cardiac cycle. More specifically, the heart ejects blood through the fully open valve in the direction of the arrow shown in FIG. 8. Shortly thereafter, the distal or downstream blood pressure starts to rise relative to the proximal pressure across the valve, creating a backpressure on the valve.

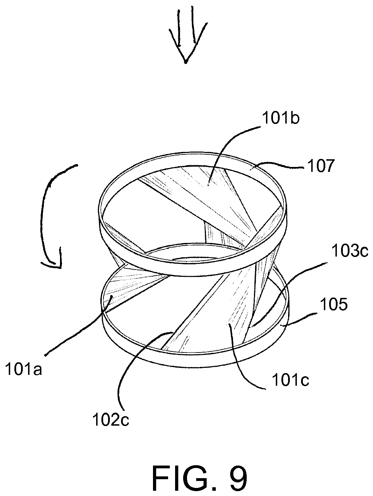

FIG. 9 is a perspective view of the valve assembly of FIG. 8 while being closed. That backpressure along the direction of the arrow causes the axially displacement of the leaflets 101 and non-stationary member 107 towards the stationary cylindrical member 105. As the leaflets 101 move from a vertical to horizontal plane relative to the longitudinal axis, a net counterclockwise torque force is exerted on the non-stationary member 107 and leaflets 101. The torque force exerts a centripetal force on the leaflets 101.

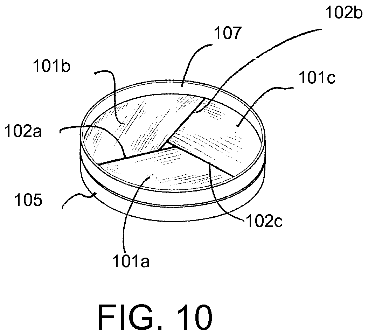

FIG. 10 is a perspective view of the valve assembly of FIG. 8 once completely closed. Complete closure of the valve 100 occurs as the leaflets 101 displace to the center of the valve and the non-stationary cylindrical member 107 rests upon the stationary member 105, as shown.

The function of the valve 100 opening can be understood by observing the reverse of the steps of valve closing, namely following the sequence of drawings from FIG. 10 to FIG. 8.

In considering the valve 100 as an aortic valve replacement, it would remain closed as shown in FIG. 10, until the heart enters systole. During systole, as the myocardium forcefully contracts, the blood pressure exerted on the valve's proximal side (the side closest to the heart) is greater than the pressure on the distal side (downstream) of the closed valve. This pressure gradient causes the leaflets 101 and non-stationary cylindrical member 107 to displace away from the stationary member 105 along the axial plane. The valve 100 briefly assumes the half-closed transition state shown in FIG. 9.

As the leaflets 101 elongate from a horizontal to vertical orientation along the axial plane, a net torque force is exerted on the leaflets 101 and non-stationary cylindrical member 107. Since the valve 100 is opening, as opposed to closing, the torque force exerted to open the valve is opposite to that exerted to close the valve. Given the configuration of embodiment shown in FIG. 9, the torque force that opens the valve would be in clockwise direction.

The torque forces cause the leaflets 101 to rotate with the non-stationary member 107 around the longitudinal axis of the valve 100. This, in turn, exerts a centrifugal force on each leaflet 101. The leaflets 101 undergo radial displacement away from the center, effectively opening the valve and allowing blood to flow away from the heart, in the direction shown by the arrow in FIG. 8.

To summarize, the valve passively functions to provide unidirectional blood flow by linking three forces. Axial, torque, and radial forces are translated in a sequential and reversible manner, while encoding the directionality of prior motions. First, the axial force of blood flow and pressure causes the displacement of the leaflets 101 and non-stationary members 107 relative to the stationary member 105 along the axial plane. This is translated into a rotational force on the leaflets 101 and non-stationary member 107. The torque force, in turn, displaces the leaflets 101 towards or away from the center of the valve, along the radial plane, which closes or opens the valve 100. The valve 100 passively follows the pathway of opening or closing, depending on the direction of the axial force initially applied to the valve by the cardiac cycle.

In the body, the stationary cylindrical member 105 can secured and fixed in position at the implant site, while the non-stationary member 107 and distal ends of leaflets 101 are free to displace along the axial plane. In using the prosthetic valve as an aortic valve replacement, the stationary member 105 would be secured in the aortic root. As the blood pressure or flow from the heart, increases, the valve 100 changes from its closed configuration to the open configuration, with blood ejecting through the valve 100.

Specific advantages of the rotating valve of FIGS. 8-10, along with further embodiments, are described in the above-incorporated parent provisional patent application.

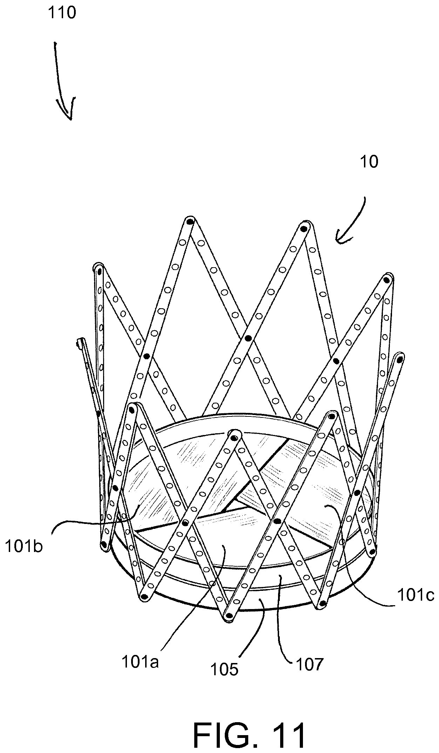

FIG. 11 is a perspective view of the valve of FIGS. 8-10 in combination with the support structure of FIG. 1. As shown in the closed position, the valve's stationary member 105 is attached to the support structure 10. The valve's nonstationary member 107 is not attached to the support structure 10. This enables the non-stationary member 107 to displace along the axial plane along with the leaflets 101 during valve opening or closing. In this particular embodiment, the valve 100 occupies a position that is closer to one end of the support structure 10, as shown.

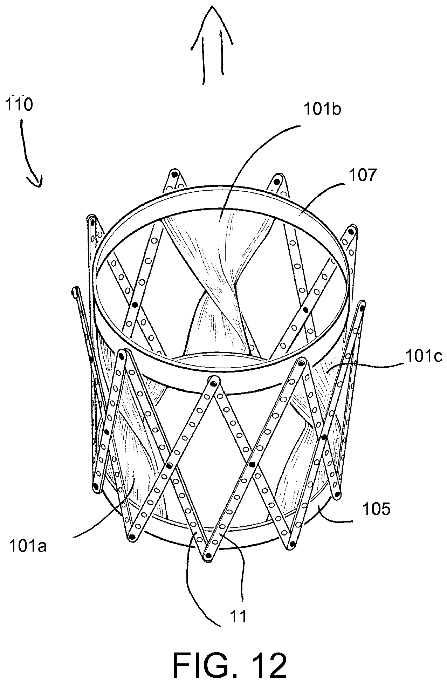

FIG. 12 is a perspective view of the valve of FIG. 11 in the open position. As noted above, the non-stationary member 107 is not attached to support structure 10, and is thus free to displace along the axial plane, along with the leaflets 101. In this particular embodiment, during full opening, non-stationary member 107 and the leaflets 101 remain within the confines of the support structure 10.

The stented valve 110 can be implanted during a closed procedure as described above. However, because of the operation of the non-stationary member within the body of the stent, the actuator mechanism to compress and expand the stent would not be disposed within the stent.

Further embodiments of the stented valve 110, positioning of the valve in the body, and procedures for implantation are described in the above-incorporated parent provisional patent application. In addition, a tissue valve can be draped on the support structure. Additional embodiments should be apparent to those of ordinary skill in the art.

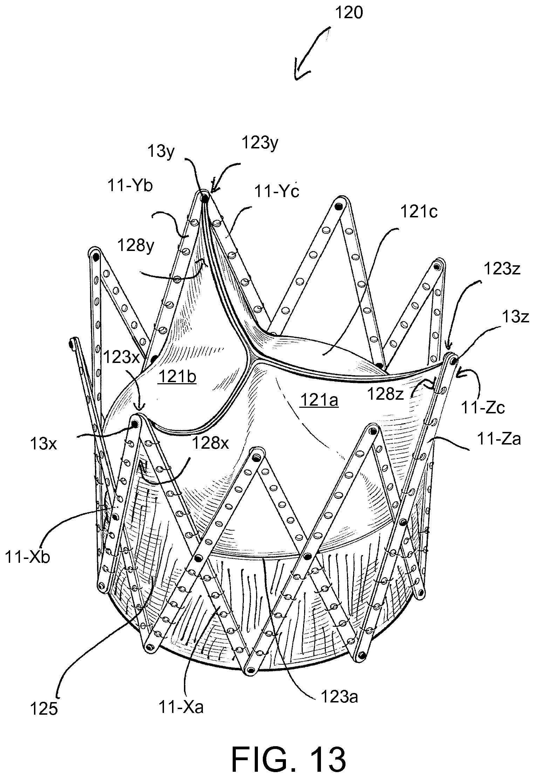

FIG. 13 is a perspective view of a traditional tissue valve mounted to the support structure of FIG. 1. As shown, a stented valve 120 includes a prosthetic tissue valve 121 attached to a support structure 10, such as that described above.

The tissue valve 121 includes three pliable semi-circular leaflets 121a, 121b, 121c, which can be derived from biocompatible materials as noted with reference to FIG. 8. Adjacent leaflets are attached in pairs to commissures 123x, 123y, 123z on the support structure 10. In particular, the commissures 123x, 123y, 123z correspond with spaced-apart distal anchor points 13x, 13y, 13z on the support structure 10. In an 18-strut stent, the commissures are attached the structure 10 via corresponding fasteners 25 at every third distal anchor point.

From the commissures, the leaflet sides are connected to the adjacent diagonal struts. That is, the sides of the first leaflet 121a are sutured to the struts 11- Xa and 11-Za, respectively; the sides of the second leaflet 121b are sutured to the struts 11-Xb and 11-Yb, respectively; and the sides of the third leaflet 121c are sutured to the struts 11-Yc and 11-Zc, respectively. Those sutures end at the scissor pivot points on the diagonal struts.

In the configuration shown, neighboring struts 11 are attached to one another in a manner that creates multiple arches 128 at the ends of the stent. Posts for leaflet attachment, or commissures, are formed by attaching neighboring leaflet to each of the struts that define a suitable arch 128x, 128y, 128z. In the configuration shown, there are three leaflets 121a, 121b, 121c, each of which is attached to a strut along two of its opposing borders. The commissures are formed by three equi-distance arches 128x, 128y, 128z in the stent.

The angled orientation of a strut in relationship to its neighboring strut enables the leaflets 121a, 121b, 121c to be attached to the stent in a triangular configuration. This triangular configuration simulates the angled attachment of the native aortic leaflet. In the native valve this creates an anatomical structure between leaflets, known as the inter-leaflet trigone. Because the anatomical inter-leaflet trigone is believed to offer structural integrity and durability to the native aortic leaflets in humans, it is advantageous to simulate this structure in a prosthetic valve.

One method of attachment of the leaflets to the struts is to sandwich the leaflet between a multi-ply strut. The multiple layers are then held together by sutures. Sandwiching the leaflets between the struts helps to dissipate the forces on leaflets and prevent the tearing of sutures through the leaflets.

The remaining side of each leaflet 121a, 121b, 121c is sutured annularly across the intermediate strut members as shown by a leaflet seam. The remaining open spaces between the struts are draped by a biocompatible skirt 125 to help seal the valve against the implant site and thus limit paravalvular leakage. As shown, the skirt 125 is shaped to cover those portions of the stent below and between the valve leaflets.

In more detail, the skirt 125 at the base of the valve is a thin layer of material that lines the stent wall. The skirt material can be pericardial tissue, polyester, PTFE, or other material or combinations of materials suitable for accepting tissue in growth, including chemically treated materials to promote tissue growth or inhibit infection. The skirt layer functions to reduce or eliminate leakage around the valve, or "paravalvular leak". To that end, there are a number of ways to attach the skirt material layer to the stent, including:
- the skirt layer can be on the inside or the outside of the stent;
- the skirt layer can occupy the lower portion of the stent;
- the skirt layer can occupy the lower and upper portion of the stent;
- the skirt layer can occupy only the upper portion of the stent;
- the skirt layer can occupy the area between the struts that define the commissure posts;
- the skirt layer can be continuous with the leaflet material;
- the skirt layer can be sutured to the struts or a multitude of sites; or
- the skirt layer can be secured to the lower portion of the stent, and pulled or pushed up to cover the outside of the stent during the deployment in the body.

The above list is not necessarily limiting as those of ordinary skill in the art may recognize alternative draping techniques for specific applications.

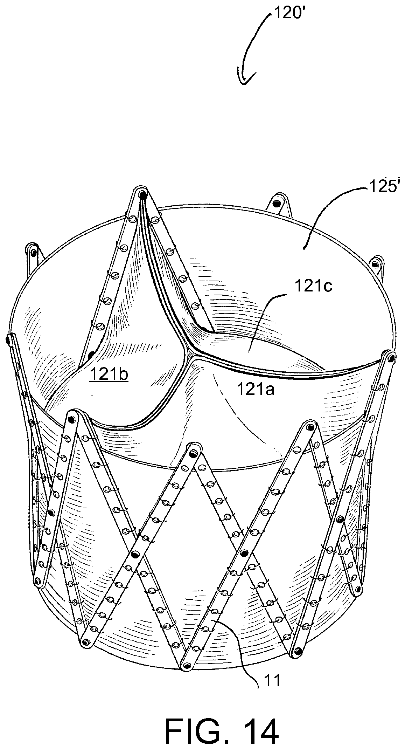

FIG. 14 is a perspective view of the valve structure of FIG. 13 having a full inner skirt. A stented valve 120' includes a prosthetic tissue valve 121' having three leaflets 121a', 121b', 121c' attached to a support structure 10. A skirt layer 125' covers the interior surface of the stent 10. As such, the valve leaflets 121a', 121b', 121c' are sutured to the skirt layer 125'.

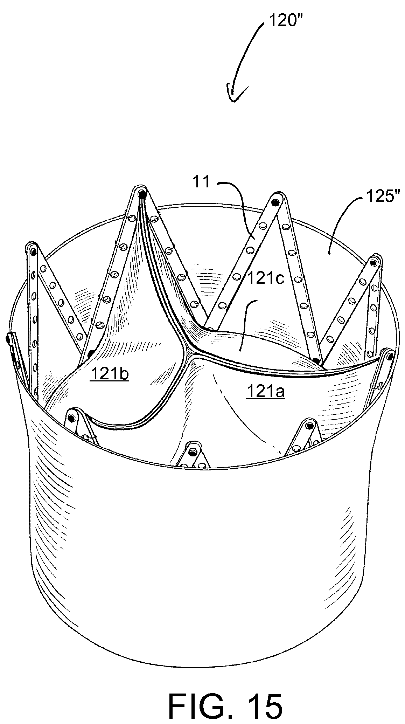

FIG. 15 is a perspective view of the valve structure of FIG. 13 having a full outer skirt. A stented valve 120" includes a prosthetic tissue valve 121" having three leaflets 121a", 121b", 121c" attached to a support structure 10, such as that described in FIG. 13. A skirt layer 125" covers the exterior surface of the stent 10.

The tissue valve structures 120, 120', 120" can also be implanted during a closed procedure as described above. However, the actuator mechanism to compress and expand the stent would be attached to avoid the commissure points and limit damage to the skirt layer 125, 125', 125", such as by mounting the actuator mechanism on the outer surface of the stent 10.

While the above-described embodiments have featured a support structure having linear strut bars and equal length scissor arms, other geometries can be employed. The resulting shape will be other than cylindrical and can have better performance in certain applications.

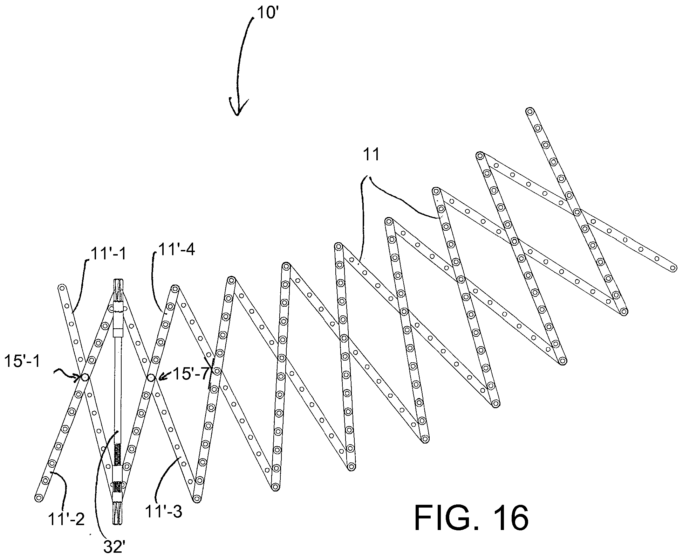

FIG. 16 is a perspective view of the arrangement of strut members in a conical-shaped support structure configuration. In the conical structure 10', the strut members 11 are arranged as shown in FIG. 2, except that the middle scissor pivots do not bisect the struts. In particular, the middle scissor pivots (e.g. 15'-1, 15'-7) divide the joined strut members (e.g. 11'-1, 11'-2 and 11'-3, 11'4) into unequal segments of 5/12 and 7/12 lengths. When fully assembled, the resulting support structure thus conforms to a conical shape when expanded. For illustration purposes, the stent 10' is shown with a single-threaded actuator rod 32' (FIG. 6), but it is not a required element for this stent embodiment.

The stent 10' can also assume a cone shape in its expanded configuration by imposing a convex or concave curvature to the individual strut members 11 that comprise the stent 10'. This could be achieved by using a material with memory, such as shape-memory or temperature sensitive Nitinol.

A valve can be orientated in the cone-shaped stent 10' such that the base of the valve was either in the narrower portion of the cone-shaped stent, with the nonbase portion of the valve in the wider portion of the cone. Alternatively, the base of the valve can be located in the widest portion of the stent with the non-base portion of the valve in the less-wide portion of the stent.

The orientation of a cone-shaped stent 10' in the body can be either towards or away from the stream of blood flow. In other body lumens (e.g. respiratory tract or gastrointestinal tract), the stent could be orientated in either direction, in relationship to the axial plane.

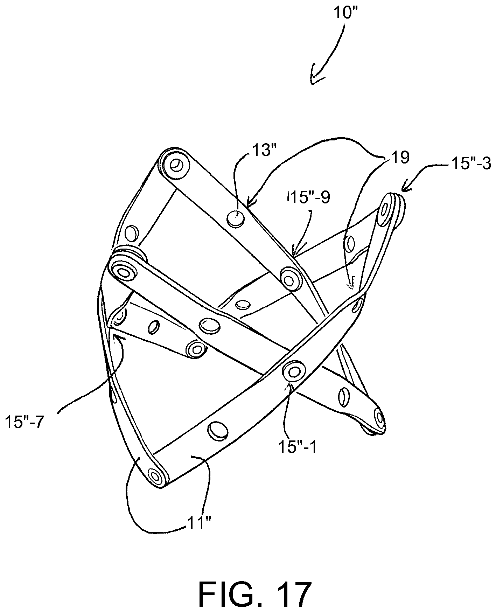

FIG. 17 is a perspective view of an hourglass-shaped support structure configuration. In this configuration, the circumference around the middle pivot points 15"-1, 15"-7, 15"-9 (the waist) is less than the circumference at either end of the stent 10". As shown, the hourglass shaped support structure 10" is achieved by reducing the number of strut members 11" to six and shortening the strut members 11" in comparison to prior embodiments. As a result of the shortening, there are fewer orifices 13" per strut member 11". Because of the strut number and geometry, each strut member 11" includes a twist at points 19" along there longitudinal planes. The twists provide a flush interface between joined strut 15"-3.

An hourglass stent configuration could also be achieved by imposing concave or convex curvatures in individual bars 11". The curvature could be a property of the materials (e.g. shape-memory or heat-sensitive Nitinol). The curvature could be absent in the compressed stent state and appear when the stent is in its expanded state.

It should be noted that any of the above-described support structures can be extended beyond the anchor joints at either of both ends of the stent. By coupling a series of stents in an end-to-end chain fashion, additional stent lengths and geometries can be fabricated. In particular, an hourglass-shaped stent could be achieved by joining two cone-shaped stents at their narrow ends. The hourglass shape can also be modified by assembling the middle scissor pivots off center as shown in FIG. 14.

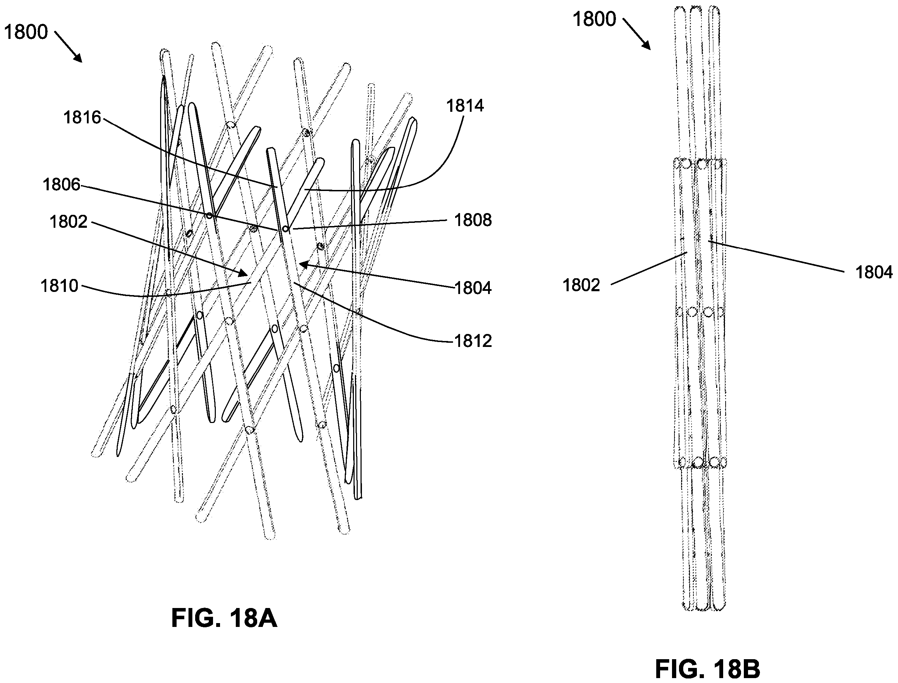

FIGS. 18A and 18B depict another embodiment of an articulated tubular structure 1800, wherein at least one of the inner and outer struts 1802, 1804 of the structure 1800 extend beyond the end articulations 1806, 1808 of the struts 1802, 1804. As depicted in FIG. 18B, when the structure 1800 is in a collapsed state, the struts 1802, 1804 have a generally linear configuration and comprising a slightly offset but generally longitudinal orientation (with respect to the longitudinal axis of the structure 1800). In the expanded stated depicted in FIG. 18A, the middle segments 1810, 1812 of the struts 1802, 1804 assume an arcuate configuration (e.g. segment of a helix) with an acute angle relative to the longitudinal axis of the structure 1800. As depicted in FIG. 18A (and also depicted in FIG. 1), in the expanded state, each of the outer struts 1816 generally comprises the same angled orientation, relative to a perimeter of the structure, transverse the longitudinal axis of the structure 1800, while each of the inner struts 1814 comprises the opposite angled orientation relative to the perimeter. The end segments 1814, 1816 of the struts 1802, 1804, however, are only supported on one end at the end articulations 1806, 1808, and therefore comprise a generally linear configuration with a tangential angle orientation relative to the adjacent middle segment 1810, 1812. The end segments 1814, 1816, overall can provide the structure 1800 with first and second perimeters that are larger than a middle perimeter of the structure 1800, e.g. a parabolic shape. The relative size differences between the first and second perimeters and the middle perimeter may be affected by the length of the end segments 1814, 1816 and the degree of expansion provided to the structure 1800, with relatively smaller size difference associated with smaller degrees of expansion and larger size differences (e.g. a more accentuated parabolic shape vs. a more cylindrical shape) at larger degrees of expansion.

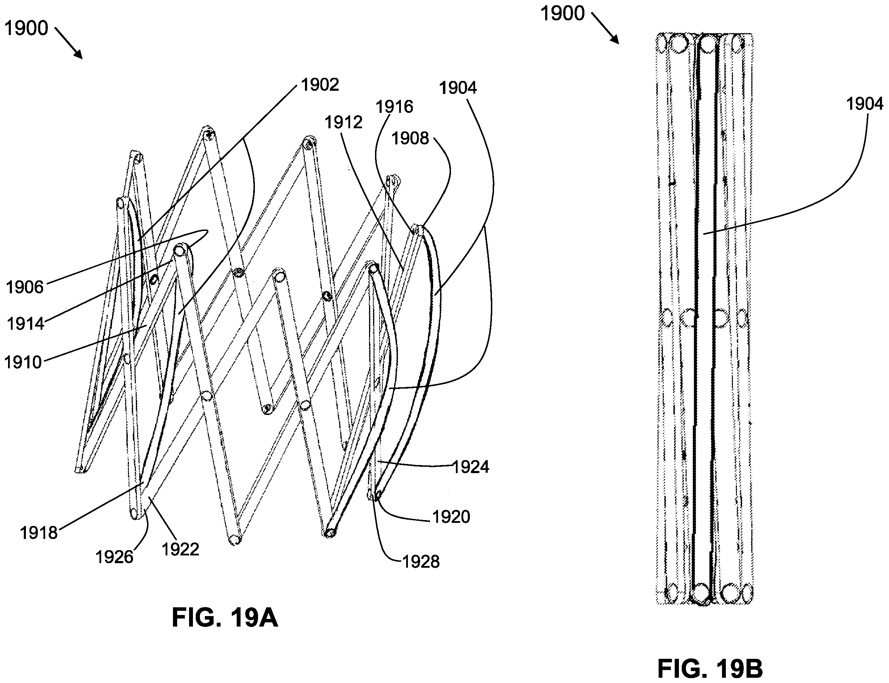

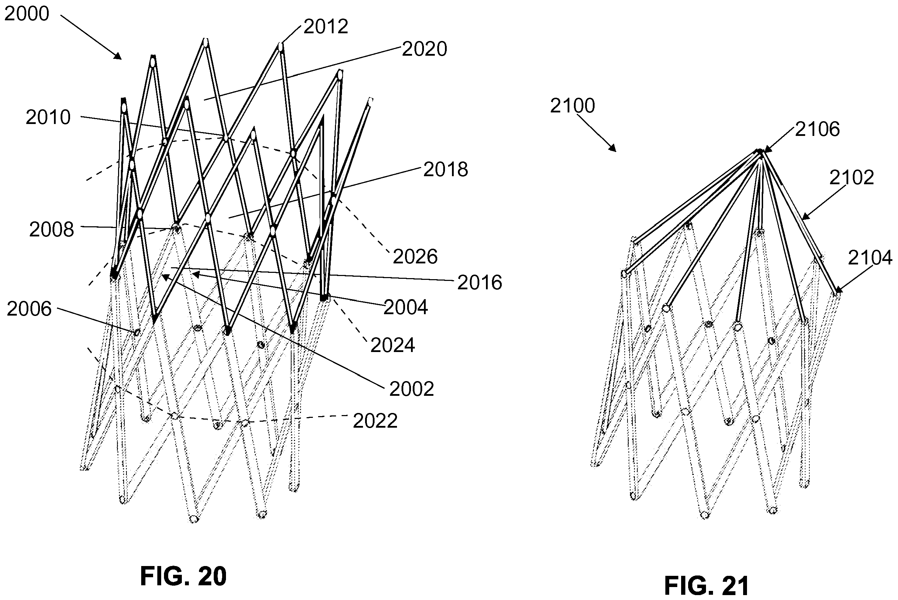

In another embodiment, illustrated in FIGS. 19A and 19B, the articulated structure 1900 may further comprise either inner 1902 and/or outer 1904 bow struts. For illustrative purposes, only selected struts 1902, 1904 are depicted on structure 1900, but a plurality of each type of bow strut 1902, 1904 is contemplated, up to every available location on the structure 1900. Each bow strut 1902, 1904 comprises a first end 1906, 1908 attached to an inner or outer strut 1910, 1912 at their respective first ends 1914, 1916, and a second end 1918, 1920 attached to a different inner or outer strut 1922, 1924 at their respective opposite ends 1926, 1928. Typically, but not always, the different inner or outer strut 1922, 1924 is immediately or directed adjacent to the original strut 1910, 1912. Alternatively, instead of being attached at the opposite ends 1926, 1928 of the different strut 1922, 1924, the bow struts may attached to a middle position or a middle articulation of the struts 1922, 1924 (including but not limited to any of the middle articulations of the multi-level articulated structure 2000 in FIG. 20, discussed below). As shown in FIG. 19B, the exemplary outer strut 1904 comprises a generally linear configuration when the structure 1900 is in a collapsed state, but in the expanded state depicted in FIG. 19A, the first end 1906, 1908 and second ends 1918, 1920 of the bow struts 1902, 1904 come closer together, causing the bow struts 1902, 1904 to bow radially inward or outward, respectively. In some variations, the bow struts 1902, 1904 may be used to circumferentially retain other structures (not shown) between the inner bow struts 1902, 1904 and the primary struts 1910, 1912, 1922, 1924. In one example, the other structure may comprise a tubular balloon, resilient seal, skirt, or elongate therapy delivery mechanism (e.g. electrodes, drug elution or drug infusion, etc.).

FIG. 20 depicts another example of an articulated structure comprising tubular articulated structure 2000 comprising longer struts 2002, 2004 with more than one middle articulation 2006, 2008, 2010, in addition to their end articulations 2012, 2014. With these additional features, the struts 2002, 2004 may be arranged to provide two or more sets of cells 2016, 2018, 2020 aligned along different perimeters 2022, 2024, 2026. In addition, to providing longer structures 2000, is was surprisingly discovered that the use of longer struts 2002, 2004 wherein at least two sets of cells 2016, 2018 are formed, the structure 2000 is capable of self-expansion without additional mechanisms or forces acting on the struts 2002, 2004 of the structure 2000. Although not wishing to be bound by the hypothesis, it is believed that this intrinsic self-expansion property of structure 2000 may be the result of greater total degree of curvature in the struts 2002, 2004 when in the collapsed state, which results in greater stress and strain acting on the struts 2002, 2004 that sufficient high that then can overcome resistance of the collapsed state to relatively straighten to the expanded state, reducing its potential energy. Even more surprising, it was found that embodiments of structure wherein the struts comprise two middle articulations and two sets of aligned cells (one each less than the embodiment depicted in FIG. 20, the structure is has an intrinsically stable collapsed state wherein the net frictional forces resisting expansion exceed expansion forces of the struts, but if the structure is slightly expanded from the collapsed state to a point where the net frictional forces of the structure are relatively lower (e.g. less overlapping surface area between the inner and outer struts), the structure still has at least some self-expansion ability. In contrast, the embodiment depicted in FIGS. 1 and 3 are configured to be inherently stable at any configuration it is placed in, e.g. full collapse, partial collapse/expansion, and full expansion.

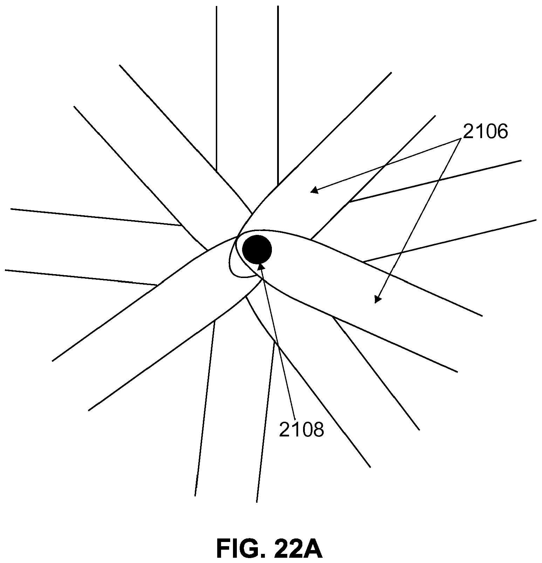

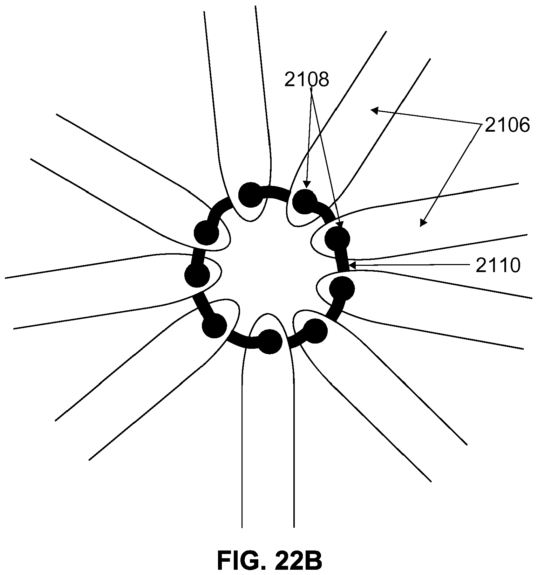

FIG. 21 schematically depicts another embodiment of an articulated structure 2100 comprising a set of radial struts 2102 with outer ends 2104 coupled to the inner and/or outer struts 2106 and inner ends 2106 coupled to the other inner ends 2106. Although FIG. 21 only depicts one set of radial struts located at one end of the structure 2100, in other embodiments, a second set of struts may be provided at the other end of the structure. The radial struts 2102 may or may not impart a radial expansion force to the inner and/or outer struts 2106, depending upon their configuration. In FIG. 22A, wherein the inner ends 2106 are rigidly affixed together, a greater expansion force may be imparted as the inner ends 2106 attempt to straighten. Although the ends 2106 in FIG. 22A are depicted with apertures 2108 on their ends 2106 that are aligned, of course, the ends 2106 may be rigidly affixed together without apertures or without aligned apertures. Furthermore, FIG. 22A depicts every radial strut attached to a single pin or attachment structure, but in other variations, multiple pins or attachment structures may be used, e.g. pairs or other subsets of the group of radial struts may be attached to their own pin or attachment structure. In contrast, in FIG. 22B, the loosely affixed ends 2106, performed by using a flexible or rigid loop or ring 2110 at their apertures 2108, may permit at least some tilting, pivoting or stress-relieving so that the radial struts do not impart any significant expansion force.

In some embodiments, a structure comprises two sets of radial struts may be used to perform radiofrequency or heater probe ablation of tissue. In further embodiments, the structure may be configured to provide circumferential ablation of tissue in a cavity or tubular body structure. Depending upon the size of the structure and the degree of radial expansion or resistance to radial collapse, the ablation structure may be selected to ablate the opening of a cavity or lumen, while resisting significant entry into the cavity or lumen by resisting collapse via the intrinsic mechanical properties of the inner and outer lumens, and/or the radial struts. Such an ablation device may be used, for example, for ablation about the pulmonary vein for treatment of cardiac arrhythmias, or for ablation about the renal artery for treatment of hypertension.

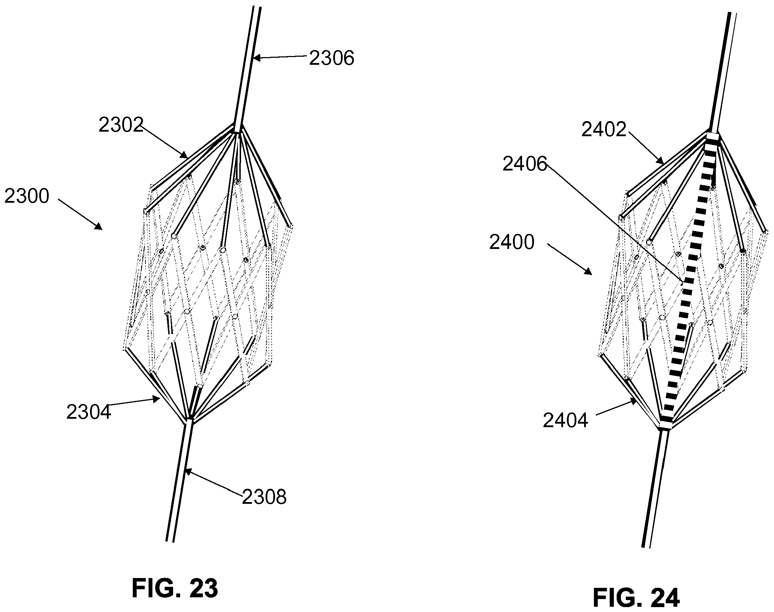

Structures comprising one or two sets of radial struts may be deployed using any of a variety of mechanisms. In FIG. 23, for example, each set of radial struts 2302 and 2304 articulate with separate deployment structures 2306 and 2308, which, depending upon whether the deployment structures 2306 and 2308 are flexible or rigid, may be configured to deployed by pulling and/or pushing of the deployment structure 2306 and 2308. In other examples, such as the structure 2400 depicted in FIG. 24, both sets of radial struts 2402 and 2404 may be attached to a common deployment structure or assembly 2406, where at least one or both of the attachments of the strut sets 2402 and 204 may be displaced longitudinally to affectuate expansion and/or collapse of the structure 2400. In some further variations, the common deployment structure or assembly 2406 may comprise a catheter with hooks or other projections configured to releasably retain the apertures of the radial struts.

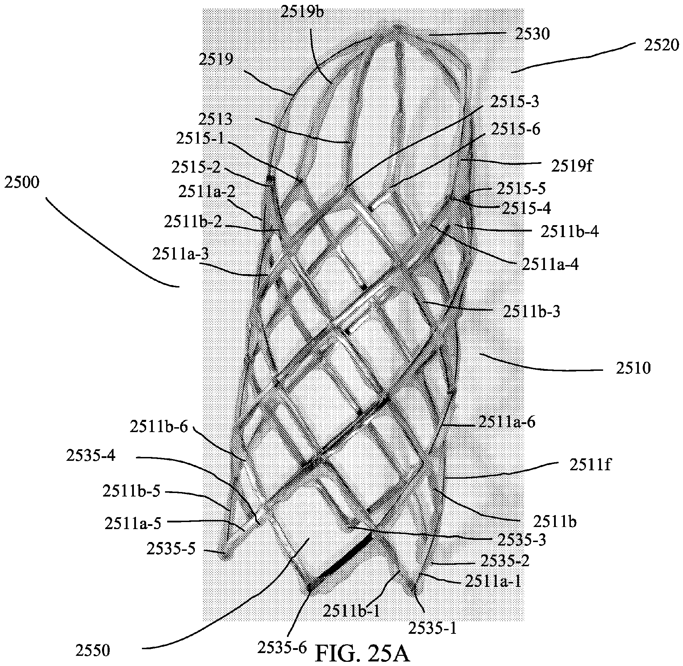

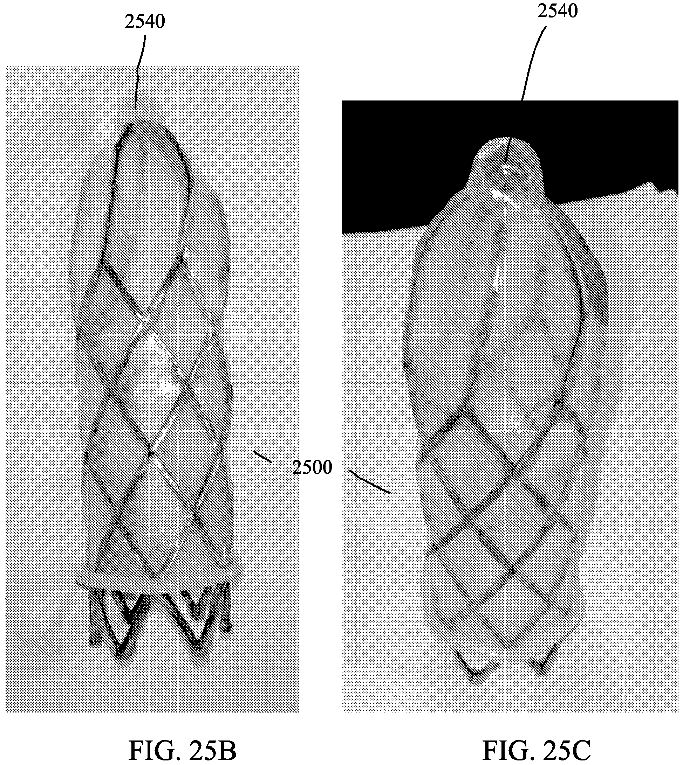

In another embodiment, shown in FIGS. 25A-C, the articulated structure may comprise a cylindrical proximal region 2510 and a closed distal region 2520. A shown, the support structure 2500 is a splint device comprising a plurality of helical strut members 2511 and a plurality of curved strut members 2519. The helical strut members 2511 and interconnected by a plurality of pivot or swivel joints to form a cylindrical structure having a central lumen 2550. The curve strut members 2519 are interconnected by one or more pivot joints to form a tapered shape, which is connected to the distal end of the cylindrical structure to form a closed end. The pivot joints allow the interconnected strut members 2511, 2519 to rotate relative to each other. As shown, there are twelve helical strut members 2511 and six curved strut members 2519. However, in other variations there may be other numbers of strut members.

The pivot or swivel joints may be interconnected by rotatable pivot fasteners, such as rivets, extending through aligned orifices, which may be further configured to permit tilting of the strut members with regards to the transverse plane of the strut rotation axis. It should be understood that other rotatable fasteners can be employed such as screws, bolts, ball-in socket structures, nails, or eyelets, and that the fasteners can be integrally formed in the struts 2511, 2519 such as a peened semi-sphere interacting with an indentation of orifice, or a male-female coupling.

The strut members 2511, 2519 may be fabricated from a rigid or semi-rigid biocompatible material, such as plastics or other polymers and metal alloys, including stainless steel, tantalum, titanium, nickel-titanium (e.g. Nitinol), and cobalt-chromium (e.g. ELGILOY). The dimensions of each strut can be chosen in accordance with its desired use. In a particular embodiment, each strut member is made from stainless steel, which is 0.005-0.020 inch thick. More particularly, each helical strut 2511 is 0.010 inch thick 300 series stainless steel, and each curve strut 2519 is 0.005 inch thick 300 series stainless steel. While all struts 2511, 2519 are shown as being of uniform thickness, the thickness of a strut can vary across a strut, such as a gradual increase or decrease in thickness along the length of a strut. Furthermore, individual struts can differ in thickness from other individual struts in the same support structure.

As shown, each strut member 2511, 2519 has front surface 2511f, 2519f, respectively, and a back surface 2511b, 2519b, respectively. In a particular embodiment, each strut member may have a width of 0.010-0.250 inches. More particularly, each helical strut 2511 is 0.050 inches wide, and each curved strut 2519 is 0.020 inches wide. The strut members can however be of different geometries. For example, instead of a uniform width, the struts can vary in width along their length. Furthermore, an individual strut can have a different width than another strut in the same support structure. Similarly, the strut lengths can vary from strut to strut within the same support structure. In a particular embodiment, each helical strut 2511 is 3-10 inches long, and each curved strut 2519 is 1.5-5 inches long. More particularly each helical strut may be 5 inches long, and each curved strut is 2 inches long. The particular dimensions may be chosen based on the intended use.

The shape of the struts can vary. The nonflatness or flatness, or degree of linearity, of the struts can be a property of the material from which they are constructed. For example, the struts can exhibit shape-memory or heat- responsive changes in shape to the struts during various states. Such states can be defined by the stent in the compressed or expanded configuration.

Furthermore, the strut members 2511, 2519 may have a smooth or rough surface texture. In particular, a pitted surface can provide tensile strength to the struts.

In certain instances, the structure may comprise struts that are multiple members stacked upon one another. Within the same structure, some struts may include elongated members stacked upon one another in a multi-ply configuration, and other struts could be one-ply, composed of single-thickness members. Within a single strut, there may be areas of one-ply and multi-ply layering of the members.

In some cases, strut members may include a plurality of orifices 2513 spaced along the length of the strut members. On the front surface, the orifices can be countersunk to receive the head of a fastener. In a particular embodiment, there are six equally spaced orifices 2513 along the length of each curved strut member 2519, but more or fewer orifices can be used. The orifices 2513 are shown as being of uniform diameter and uniform spacing along the strut member 2519, but neither is required. No orifices are shown on helical strut members 2511, but helical strut members 2511 may have orifices 2513 or recesses along their length.

The helical strut members 2511 may be arranged in a cylindrical shape 2510, and the curved strut members 2519 are arranged in a tapered shape 2520. The proximal end of the tapered shape 2520 may be swivelably connected to the distal end of the cylindrical shape 2510 to create a closed distal end of structure 2500. In other variations, the structure 2500 may have an open distal end. In other variations, the struts may be continuous between the cylindrical shape 2510 and the curved shape 2520.

The structure 2500 may be configured such that it can act as a splinting device for a portion of the body. In particular, the splint device 2500 may be configured to be placed around the penis. In other variations, the splint device may be configured to be placed around other portions of the body, such as an arm, leg, finger, or toe.

As shown in FIGS. 25A-C, cylindrical shape 2510 comprises right- and left-handed helical strut members 2511. Each right-handed strut member 2511a may be an outer strut member. Each left-handed helical strut member 2511b may be an inner strut member. Each outer, right-handed individual strut member 2511a may be rotatably connected to an individual inner, left-handed strut member 2511b strut member, with their back surfaces in oriented toward each other.

In particular, each outer, right-handed strut member 2511a may be rotatably connected to an inner, left-handed strut member 2511b by a distal anchor pin joint 2515, located near the distal ends of the strut members 2511a, 2511b, and a proximal anchor pin joint 2935, located near the proximal ends of the strut members 2511a, 2511b. Each outer, right-handed strut member 2511a may also be rotatably connected to each of the five remaining inner, left-handed strut members 2511b via a scissor pin joint 2955.

More specifically, outer, right-handed strut member 2511a-1 may be rotatably connected to inner, left-handed strut member 2511b-1 by a distal anchor pin joint 2515-1, located near the distal ends of the strut members 2511a-1, 2511b-1. Outer, right-handed strut member 2511a-1 may be also rotatably connected to inner, left-handed strut member 2511b-1 by a proximal anchor pin joint 2535-1, located near the distal ends of the strut members 2511a-1, 2511b-1.

In addition, proximal to pin joint 2515-1, outer, right-handed strut member 2511a-1 may be rotatably connected via scissor pin joint 2955 to inner, left-handed strut member 2511b-6. Proximal to its connection with strut member 2511b-6, strut member 2511a-1 may be rotatably connected via scissor pin joint 2555 to inner, left-handed strut member 2511b-5. Proximal to its connection with strut member 2511b-5, strut member 2511a-1 may be rotatably connected via scissor pin joint 2555 to inner, left-handed strut member 2511b-4. Proximal to its connection with strut member 2511b-4, strut member 2511a-1 may be rotatably connected via scissor pin joint 2555 to inner, left-handed strut member 2511b-3. Proximal to its connection with strut member 2511b-3, strut member 2511a-1 may be rotatably connected via scissor pin joint 2555 to inner, left-handed strut member 2511b-2. Proximal to the connection between strut members 2511a-1 and 2511b-2 may be the proximal anchor pin joint 2935-1.

In addition, proximal to pin joint 2515-1, inner, left-handed strut member 2511b-1 may be rotatably connected via scissor pin joint 2555 to outer, right-handed strut member 2511a-2. Proximal to its connection with strut member 2511a-2, strut member 2511b-1 may be rotatably connected via scissor pin joint 2555 to outer, right-handed strut member 2911a-3. Proximal to its connection with strut member 2511a-3, strut member 2511b-1 may be rotatably connected via scissor pin joint 2555 to outer, right-handed strut member 2511a-4. Proximal to its connection with strut member 2511a-4, strut member 2511b-1 may be rotatably connected via scissor pin joint 2555 to outer, right-handed strut member 2511a-5. Proximal to its connection with strut member 2511a-5, strut member 2511b-1 may be rotatably connected via scissor pin joint 2555 to outer, right-handed strut member 2511a-6. Proximal to the connection between strut members 2511b-1 and 2511a-6 is the proximal anchor pin joint 2935-1.

Similar patterns of articulations exist between the remaining outer, right-handed strut members 2511a and inner, left-handed strut members 2511b. More specifically, outer, right-handed strut member 2511a-2 may be rotatably connected to inner, left-handed strut member 2511b-2 by a distal anchor pin joint 2515-2, located near the distal ends of the strut members 2511a-2, 2511b-2. Outer, right-handed strut member 2511a-2 may also be rotatably connected to inner, left-handed strut member 2511b-2 by a proximal anchor pin joint 2935-2, located near the distal ends of the strut members 2511a-2, 2511b-2.

In addition, proximal to pin joint 2515-2, outer, right-handed strut member 2511a-2 may be rotatably connected via scissor pin joint 2555 to inner, left-handed strut member 2511b-1. Proximal to its connection with strut member 2511b-1, strut member 2511a-2 may be rotatably connected via scissor pin joint 2555 to inner, left-handed strut member 2511b-6. Proximal to its connection with strut member 2511b-6, strut member 2511a-2 may be rotatably connected via scissor pin joint 2555 to inner, left-handed strut member 2511b-5. Proximal to its connection with strut member 2511b-5, strut member 2511a-2 may be rotatably connected via scissor pin joint 2555 to inner, left-handed strut member 2511b-4. Proximal to its connection with strut member 2511b-4, strut member 2511a-2 may be rotatably connected via scissor pin joint 2555 to inner, left-handed strut member 2511b-3. Proximal to the connection between strut members 2511a-2 and 2511b-3 may be the proximal anchor pin joint 2535-2.

In addition, proximal to pin joint 2515-2, inner, left-handed strut member 2511b-2 may be rotatably connected via scissor pin joint 2955 to outer, right-handed strut member 2511a-3. Proximal to its connection with strut member 2511a-3, strut member 2511b-2 may be rotatably connected via scissor pin joint 2555 to outer, right-handed strut member 2511a-4. Proximal to its connection with strut member 2511a-4, strut member 2511b-2 may be rotatably connected via scissor pin joint 2555 to outer, right-handed strut member 2511a-5. Proximal to its connection with strut member 2511a-5, strut member 2511b-2 may be rotatably connected via scissor pin joint 2555 to outer, right-handed strut member 2511a-6. Proximal to its connection with strut member 2511a-6, strut member 2511b-2 may be rotatably connected via scissor pin joint 2555 to outer, right-handed strut member 2511a-1. Proximal to the connection between strut members 2511b-2 and 2511a-1 may be the proximal anchor pin joint 2535-2.

Outer, right-handed strut member 2511a-3 may be rotatably connected to inner, left-handed strut member 2511b-3 by a distal anchor pin joint 2515-3, located near the distal ends of the strut members 2511a-3, 2511b-3. Outer, right-handed strut member 2511a-3 may also be rotatably connected to inner, left-handed strut member 2511b-3 by a proximal anchor pin joint 2535-3, located near the distal ends of the strut members 2511a-3, 2511b-3.

In addition, proximal to pin joint 2515-3, outer, right-handed strut member 2511a-3 may be rotatably connected via scissor pin joint 2555 to inner, left-handed strut member 2511b-2. Proximal to its connection with strut member 2511b-2, strut member 2511a-3 may be rotatably connected via scissor pin joint 2555 to inner, left-handed strut member 2511b-1. Proximal to its connection with strut member 2511b-1, strut member 2511a-3 is rotatably connected via scissor pin joint 2555 to inner, left-handed strut member 2511b-6. Proximal to its connection with strut member 2511b-6, strut member 2511a-3 may be rotatably connected via scissor pin joint 2555 to inner, left-handed strut member 2511b-5. Proximal to its connection with strut member 2511b-5, strut member 2511a-3 may be rotatably connected via scissor pin joint 2555 to inner, left-handed strut member 2511b-4. Proximal to the connection between strut members 2511a-3 and 2511b-4 may be the proximal anchor pin joint 2935-3.

In addition, proximal to pin joint 2515-3, inner, left-handed strut member 2511b-3 is rotatably connected via scissor pin joint 2555 to outer, right-handed strut member 2511a-4. Proximal to its connection with strut member 2511a-4, strut member 2511b-3 may be rotatably connected via scissor pin joint 2555 to outer, right-handed strut member 2511a-5. Proximal to its connection with strut member 2511a-5, strut member 2511b-3 may be rotatably connected via scissor pin joint 2555 to outer, right-handed strut member 2511a-6. Proximal to its connection with strut member 2511a-6, strut member 2511b-3 may be rotatably connected via scissor pin joint 2955 to outer, right-handed strut member 2511a-1. Proximal to its connection with strut member 2511a-1, strut member 2511b-3 may be rotatably connected via scissor pin joint 2555 to outer, right-handed strut member 2511a-2. Proximal to the connection between strut members 2511b-3 and 2511a-2 is the proximal anchor pin joint 2535-3.

Outer, right-handed strut member 2511a-4 may be rotatably connected to inner, left-handed strut member 2511b-4 by a distal anchor pin joint 2515-4, located near the distal ends of the strut members 2511a-4, 2511b-4. Outer, right-handed strut member 2511a-4 may also be rotatably connected to inner, left-handed strut member 2511b-4 by a proximal anchor pin joint 2535-4, located near the distal ends of the strut members 2511a-4, 2511b-4.

In addition, proximal to pin joint 2515-4, outer, right-handed strut member 2511a-4 may be rotatably connected via scissor pin joint 2555 to inner, left-handed strut member 2511b-3. Proximal to its connection with strut member 2511b-3, strut member 2511a-4 is rotatably connected via scissor pin joint 2555 to inner, left-handed strut member 2511b-2. Proximal to its connection with strut member 2511b-2, strut member 2511a-4 may be rotatably connected via scissor pin joint 2555 to inner, left-handed strut member 2511b-1. Proximal to its connection with strut member 2511b-1, strut member 2511a-4 may be rotatably connected via scissor pin joint 2555 to inner, left-handed strut member 2511b-6. Proximal to its connection with strut member 2511b-6, strut member 2511a-4 may be rotatably connected via scissor pin joint 2555 to inner, left-handed strut member 2511b-5. Proximal to the connection between strut members 2511a-4 and 2511b-5 may be the proximal anchor pin joint 2935-4.

In addition, proximal to pin joint 2515-4, inner, left-handed strut member 2511b-4 may be rotatably connected via scissor pin joint 2555 to outer, right-handed strut member 2511a-5. Proximal to its connection with strut member 2511a-5, strut member 2511b-4 may be rotatably connected via scissor pin joint 2555 to outer, right-handed strut member 2511a-6. Proximal to its connection with strut member 2511a-6, strut member 2511b-4 may be rotatably connected via scissor pin joint 2555 to outer, right-handed strut member 2511a-1. Proximal to its connection with strut member 2511a-1, strut member 2511b-4 may be rotatably connected via scissor pin joint 2555 to outer, right-handed strut member 2511a-2. Proximal to its connection with strut member 2511a-2, strut member 2511b-4 may be rotatably connected via scissor pin joint 2555 to outer, right-handed strut member 2511a-3. Proximal to the connection between strut members 2511b-4 and 2511a-3 may be the proximal anchor pin joint 2535-4.

Outer, right-handed strut member 2511a-5 may be rotatably connected to inner, left-handed strut member 2511b-5 by a distal anchor pin joint 2515-5, located near the distal ends of the strut members 2511a-5, 2511b-5. Outer, right-handed strut member 2511a-5 may also be rotatably connected to inner, left-handed strut member 2511b-5 by a proximal anchor pin joint 2535-5, located near the distal ends of the strut members 2511a-5, 2511b-5.

In addition, proximal to pin joint 2515-5, outer, right-handed strut member 2511a-5 may be rotatably connected via scissor pin joint 2555 to inner, left-handed strut member 2511b-4. Proximal to its connection with strut member 2511b-4, strut member 2511a-5 may be rotatably connected via scissor pin joint 2555 to inner, left-handed strut member 2511b-3. Proximal to its connection with strut member 2511b-3, strut member 2511a-5 may be rotatably connected via scissor pin joint 2555 to inner, left-handed strut member 2511b-2. Proximal to its connection with strut member 2511b-2, strut member 2511a-5 is rotatably connected via scissor pin joint 2555 to inner, left-handed strut member 2511b-1. Proximal to its connection with strut member 2511b-1, strut member 2511a-5 may be rotatably connected via scissor pin joint 2555 to inner, left-handed strut member 2511b-6. Proximal to the connection between strut members 2511a-5 and 2511b-6 may be the proximal anchor pin joint 2535-5.

In addition, proximal to pin joint 2515-5, inner, left-handed strut member 2511b-5 may be rotatably connected via scissor pin joint 2555 to outer, right-handed strut member 2511a-6. Proximal to its connection with strut member 2511a-6, strut member 2511b-5 may be rotatably connected via scissor pin joint 2555 to outer, right-handed strut member 2511a-1. Proximal to its connection with strut member 2511a-1, strut member 2511b-5 may be rotatably connected via scissor pin joint 2555 to outer, right-handed strut member 2511a-2. Proximal to its connection with strut member 2511a-2, strut member 2511b-5 may be rotatably connected via scissor pin joint 2555 to outer, right-handed strut member 2511a-3. Proximal to its connection with strut member 2511a-3, strut member 2511b-5 is rotatably connected via scissor pin joint 2555 to outer, right-handed strut member 2511a-4. Proximal to the connection between strut members 2511b-5 and 2511a-4 may be the proximal anchor pin joint 2935-5.

Outer, right-handed strut member 2511a-6 is rotatably connected to inner, left-handed strut member 2511b-6 by a distal anchor pin joint 2515-6, located near the distal ends of the strut members 2511a-6, 2511b-6. Outer, right-handed strut member 2511a-6 is also rotatably connected to inner, left-handed strut member 2511b-6 by a proximal anchor pin joint 2535-6, located near the distal ends of the strut members 2511a-6, 2911b-6.

In addition, proximal to pin joint 2515-6, outer, right-handed strut member 2511a-6 is rotatably connected via scissor pin joint 2555 to inner, left-handed strut member 2511b-5. Proximal to its connection with strut member 2511b-5, strut member 2511a-6 is rotatably connected via scissor pin joint 2555 to inner, left-handed strut member 2511b-4. Proximal to its connection with strut member 2511b-4, strut member 2511a-6 is rotatably connected via scissor pin joint 2555 to inner, left-handed strut member 2511b-3. Proximal to its connection with strut member 2511b-3, strut member 2511a-6 is rotatably connected via scissor pin joint 2555 to inner, left-handed strut member 2511b-2. Proximal to its connection with strut member 2511b-2, strut member 2511a-6 is rotatably connected via scissor pin joint 2555 to inner, left-handed strut member 2511b-1. Proximal to the connection between strut members 2511a-6 and 2511b-1 is the proximal anchor pin joint 2535-6.

In addition, proximal to pin joint 2515-6, inner, left-handed strut member 2511b-6 is rotatably connected via scissor pin joint 2555 to outer, right-handed strut member 2511a-1. Proximal to its connection with strut member 2511a-1, strut member 2511b-6 is rotatably connected via scissor pin joint 2555 to outer, right-handed strut member 2511a-2. Proximal to its connection with strut member 2511a-2, strut member 2511b-6 is rotatably connected via scissor pin joint 2555 to outer, right-handed strut member 2511a-3. Proximal to its connection with strut member 2511a-3, strut member 2511b-6 is rotatably connected via scissor pin joint 2555 to outer, right-handed strut member 2511a-4. Proximal to its connection with strut member 2511a-4, strut member 2511b-6 is rotatably connected via scissor pin joint 2555 to outer, right-handed strut member 2511a-5. Proximal to the connection between strut members 2511b-6 and 2511a-5 is the proximal anchor pin joint 2535-6.

Curved strut members 2519 are arranged to form the closed tapered distal region 2520. The six curved strut members 2519 are interconnected at their distal end by a pivot joint 2565 using rotatable pivot faster, such as a rivet. It should be understood that other rotatable fasteners can be employed such as screws, bolts, ball-in-socket structures, nails, or eyelets, and that the fasteners can be integrally formed in the struts 2519 such as a peened semi-sphere interacting with an indentation or orifice, or a male-female coupling. Each curved strut member 2519 may be connected at its proximal end to the distal ends of two interconnected helical struts 2511. It should be understood that tapered distal region 2520 can also comprise other numbers of curved strut members. In one variation, the tapered distal region may comprise six curved strut members, wherein each curved strut member is connected at both ends to the distal end of cylindrical region 2520, and the six curved strut members are interconnected by a pivot joint bisecting the length of each curved strut member.

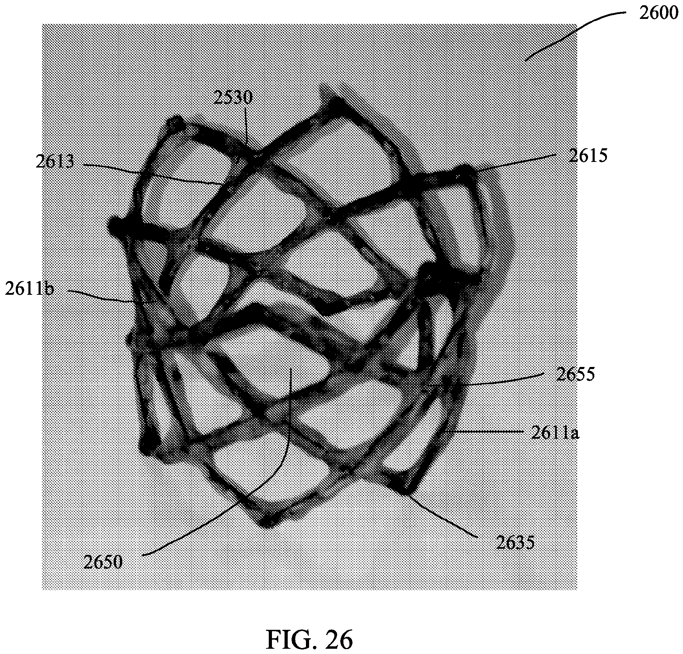

In other embodiments, the device of FIGS. 25A-C may be modified to comprise only the proximal portion of the cylindrical region 2510 of device 2500, forming a ring structure 2600 having a cylindrical shape and central lumen 2650, as shown in FIG. 26. As shown, the ring structure 2600 may have six right-handed, outer helical strut members 2611a and six left-handed, inner helical strut members 2611b, but in other variations there may be other four, five, seven, eight, nine, ten or more of each helical strut member. Each right-handed helical strut member 2611a may be rotatably connected at its distal end to a left-handed helical strut member 2611b at anchor pivot joint 2615, and each right-handed helical strut member 2611a may be rotatably connected at its proximal end to another left-handed helical strut member 2611b at anchor pivot joint 2635. Between anchor pivot joints 2615 and 2635, each right-handed helical strut member 2611a may be rotatably connected at three pivot joints 2655 to three left-handed helical strut members 2611b. Between anchor pivot joints 2615 and 2635, each left-handed helical strut member 2611b may be rotatably connected to three pivot joints 2655 to three right-handed helical strut members.

FIG. 26 also shows a variation in which helical strut members 2611 comprises a plurality of orifices 2613 spaced along the length of the strut members 2611. On the front surface, the orifices can be countersunk to receive the head of a fastener. In the embodiment in FIG. 26, there are ten equally spaced orifices 2613 along the length of each curved strut member 2611, but more or fewer orifices can be used. The orifices 2613 are shown as being of uniform diameter and uniform spacing along the strut member 2611, but neither is required. There are two open orifices 2613 in between each pivot joint along the length of each strut member 2611, but there can be more or fewer orifices between joints.

The splint structure 2500 and ring structure 2600 can be expanded or compressed by actuating the linkages to open or close the links. The structures 2500, 2600 can be reversibly expanded and compressed. The proximal region 2510 and closed tapered region 2520 of structure 2500 are connected such that by actuating the linkages in one region, the full structure 2500 is expanded or compressed. The structures 2500, 2600 can be axially collapsed, and then expanded for placement over the penis or other body part. The diameter of structures 2500, 2600 can be chosen based on the intended use. In a particular embodiment for placement over the penis, in one variation, the diameter may be 1-2 inches. More particularly, the diameter may be 1.5 inches.

As shown in FIGS. 25A-C and 26, the splint device 2500 and ring structure 2600 may comprise a polymer coating 2530. As shown, the polymer is latex, but in other embodiments it may be other polymers, such as vinyl. The polymer coating may, as shown in FIGS. 25A-C and 26, be on both the inside and outside surfaces of the structures 2500, 2600. In other embodiments, it may be only on the outside surface, or only on the inside surface. In addition to serving a protective function for tissue coming in contact with the structures 2500, 2600, the polymer coating 2530 can also make the structures 2500, 2600 self-expanding by exerting a force causing the structures 2500, 2600 to expand when the structures are in a collapsed state. In other examples, a woven or braided cover may be provided over each strut, or the splint device 2500 in the aggregate, and may comprise materials such as PTFE or ePTFE, polyester, polyethylene, and the like, as well as blends, copolymers and block copolymers thereof.

The splint device 2500 may also contain a constriction element 2540. FIGS. 25B-C show a constriction element 2540 in the form of a condom placed over splint device 2500. The constriction element 2540 can create radially inward constrictive pressure. In the case of placement over a penis, the constrictive pressure may maintain a greater volume of blood within the penis by reducing the flow of blood out of the penis. It should be recognized that the constriction element 2540 may take other forms, such as an elastic ring. The ring structure 2600 may similarly include a constriction element 2640 to create a radially inward constrictive pressure. The constrictive element 2640 may be a condom, elastic ring, or other form.

Particular embodiments of the invention offer distinct advantages over the prior art, including in their structure and applications. While certain advantages are summarized below, the summary is not necessarily a complete list as there may be additional advantages.

The device allows the user to advert the serious complications that can occur during percutaneous heart valve implantation. Because the device is retrievable and re-positionable during implantation into the body, the surgeon can avoid serious complications due to valve malpositioning or migration during implantation. Examples of these complications include occlusion of the coronary arteries, massive paravalvular leakage, or arrthymias.

The device can also decrease vascular access complications because of the device's narrow insertion profile. The device's profile is low, in part, due to its unique geometry, which allows neighboring struts in the stent to overlap during stent compression. The device's low profile is further augmented by eliminating the necessity for a balloon or a sheath. The device's narrow profile offers the advantage of widening the vascular access route options in patients. For instance, the device can enable the delivery of the prosthetic valve through an artery in the leg in a patient whom would have previously been committed to a more invasive approach through the chest wall. The device therefore aims to decrease complications associated with the use of large profile devices in patients with poor vascular access.

The tissue valve embodiments can offer improved durability by allowing for attachment of the leaflets to flexible commissural posts. The flexible posts allow dissipation of the stress and strain imposed on the leaflet by the cardiac cycle. The use of multi-ply struts enables the leaflets to be sandwiched in between the struts, which re-enforces the leaflet attachments and prevents tearing of sutures. The valve further assumes a desirable leaflet morphology, which further reduces the stress and strain on leaflets. Namely, the angled leaflet attachment to the stent is similar to the native human aortic valve's inter-leaflet trigone pattern. These properties significantly improve the longevity of percutaneous heart valve replacement therapies.

The device could reduce or eliminate arrthymia complications due to the incremental expansion or compression of the stent. The stent can employ a screw mechanism for deployment, which enables the stent to self-lock or un-lock at all radii. This enables more controlled deployment and the potential for individualizing the expansion or compression of the device in each patient. Because the expansion or compression of the device is reversible at any stage during the procedure, the surgeon can easily reverse the expansion of the device to relieve an arrhythmia. In addition, if an arrhythmia is detected during implantation, the device can be repositioned to further eliminate the problem.

The device can reduce or eliminate paravalvular leak due to the device's ability to be accurately positioned, and re-positioned, if necessary. That can considerably decrease the occurrence and severity of paravalular leaks.

The device eliminates balloon-related complications. The screw mechanism of deployment exploits the mechanical advantage of a screw. This provides for forceful dilation of the stent. The lever arms created by the pivoting of the struts in the scissor linkage of the stent, transmits a further expansion force to the stent. The stent is expanded without the need for a balloon. In addition, the ability of the device to be forcefully dilated reduces or eliminates the need for pre- or postballooning during the implantation procedure in patients.

The device has more predictable and precise positioning in the body because the difference between the height of the stent in the compressed and expanded position is small. This "reduced foreshortening" helps the surgeon to position the device in the desirable location in the body. The ability to re-position the device in the body further confers the ability to precisely position the device in each individual.

In addition to the mechanical advantages, the device enables a wider population of patients to be treated by a less invasive means for valve replacement. For example, the device enables patients with co-morbidities, whom are not candidates for open chest surgical valve replacement, to be offered a treatment option. The device's ability to assume a narrow profile also enables patients who were previously denied treatment due to poor vascular access (e.g. tortuous, calcified, or small arteries), to be offered a treatment option. The durability of the valve should expand the use of less-invasive procedures to the population of otherwise healthy patients, whom would otherwise be candidates for open chest surgical valve replacement. The device's ability to be forcefully expanded, or assume hourglass, or conical shapes, potentially expands the device application to the treatment of patients diagnosed with aortic insufficiency, as well as aortic stenosis.

The device can also provide a less invasive treatment to patients with degenerative prosthesis from a prior implant, by providing for a "valve-in-valve" procedure. The device could be accurately positioned inside the failing valve, without removing the patient's degenerative prosthesis. It would help the patient by providing a functional valve replacement, without a "redo" operation and its associated risks.

While this invention has been particularly shown and described with references to particular embodiments, it will be understood by those skilled in the art that various changes in form and details may be made to the embodiments without departing from the scope of the invention encompassed by the appended claims. For the methods disclosed herein, the steps need not be performed sequentially. Each of the features depicted in each embodiment herein in may be adapted for use in other embodiments herein.

## Claims

1. A biocompatible articulated support structure, comprising:
a tubular structure (1800), comprising:
a central lumen;
a central axis;
a plurality of discrete inner struts (1802); and
a plurality of discrete outer struts (1804);
wherein each of the plurality of discrete inner struts and the plurality of discrete outer struts comprises a first end, a second end, and a net length therebetween;
wherein each of the plurality of discrete inner struts comprises articulations (1806, 1808) with at least three different discrete outer struts of the plurality of discrete outer struts;
wherein each of the plurality of discrete outer struts comprises articulations with at least three different discrete inner struts of the plurality of discrete inner struts;
wherein no discrete inner strut of the plurality of discrete inner struts comprises articulations with any other discrete inner strut of the plurality of discrete inner struts; and
wherein no discrete outer strut of the plurality of discrete outer struts comprises articulations with any other discrete outer strut of the plurality of discrete outer struts;
**characterized in that** the first end of each of the plurality of discrete inner struts is spaced apart from a closest articulation by about at least 25% of the net length of that strut and the second end of each of said plurality of discrete inner struts is spaced apart from a closest articulation by about at least 25% of the net length of that strut.

2. The biocompatible articulated support structure of claim 1, wherein the first end of each of the plurality of discrete outer struts (1804) is spaced apart from a closest articulation by about at least 25% of its net length.

3. The biocompatible articulated support structure of claim 2, wherein the second end of each of the plurality of discrete outer struts (1804) is spaced apart from a closest articulation by about at least 25% of its net length.

## Patentansprüche

1. Biokompatible gelenkige Tragestruktur, umfassend:
eine röhrenförmige Struktur (1800), umfassend:
ein mittleres Lumen;
eine Mittelachse
eine Vielzahl separater Innenstreben (1802); und
eine Vielzahl separater Außenstreben (1804);
wobei jede der Vielzahl separater Innenstreben und der Vielzahl separater Außenstreben ein erstes Ende, ein zweites Ende und eine Nettolänge dazwischen umfasst;
wobei jede der Vielzahl separater Innenstreben Gelenke (1806, 1808) mit mindestens drei verschiedenen separaten Außenstreben der Vielzahl separater Außenstreben umfasst;
wobei jede der Vielzahl separater Außenstreben Gelenke mit mindestens drei verschiedenen separaten Innenstreben der Vielzahl separater Innenstreben umfasst;
wobei keine separate Innenstrebe der Vielzahl separater Innenstreben Gelenke mit einer anderen separaten Innenstrebe der Vielzahl separater Innenstreben umfasst; und
wobei keine separate Außenstrebe der Vielzahl separater Außenstreben Gelenke mit einer anderen separaten Außenstrebe der Vielzahl separater Außenstreben umfasst;
**dadurch gekennzeichnet, dass** das erste Ende jeder der Vielzahl separater Innenstreben von dem nächsten Gelenk ungefähr mindestens 25 % der Nettolänge dieses Gelenks und das zweite Ende jeder der Vielzahl separater Innenstreben von dem nächsten Gelenk ungefähr mindestens 25 % der Nettolänge dieses Gelenks entfernt ist.

2. Biokompatible gelenkige Tragestruktur nach Anspruch 1, wobei das erste Ende jeder der Vielzahl separater Außenstreben (1804) ungefähr mindestens 25 % ihrer Nettolänge von dem nächsten Gelenk entfernt ist.

3. Biokompatible gelenkige Tragestruktur nach Anspruch 2, wobei das zweite Ende jeder der Vielzahl separater Außenstreben (1804) ungefähr mindestens 25 % ihrer Nettolänge von dem nächsten Gelenk entfernt ist.

## Revendications

1. Structure de support articulée biocompatible, comprenant :
une structure tubulaire (1800), comprenant :
une lumière centrale ;
un axe central ;
une pluralité d'entretoises intérieures distinctes (1802) ; et
une pluralité d'entretoises extérieures distinctes (1804) ;
dans laquelle chacune parmi la pluralité d'entretoises intérieures distinctes et la pluralité d'entretoises extérieures distinctes comprend une première extrémité, une seconde extrémité, et une longueur nette entre celles-ci ;
dans laquelle chacune parmi la pluralité d'entretoises intérieures distinctes comprend des articulations (1806, 1808) avec au moins trois différentes entretoises extérieures distinctes parmi la pluralité d'entretoises extérieures distinctes ;
dans laquelle chacune parmi la pluralité d'entretoises extérieures distinctes comprend des articulations avec au moins trois différentes entretoises intérieures distinctes parmi la pluralité d'entretoises intérieures distinctes ;
dans laquelle aucune entretoise intérieure distincte parmi la pluralité d'entretoises intérieures distinctes ne comprend d'articulations avec une quelconque autre entretoise intérieure distincte parmi la pluralité d'entretoises intérieures distinctes ; et
dans laquelle aucune entretoise extérieure distincte parmi la pluralité d'entretoises extérieures distinctes ne comprend d'articulations avec une quelconque autre entretoise extérieure distincte parmi la pluralité d'entretoises extérieures distinctes ;
**caractérisée en ce que** la première extrémité de chacune parmi la pluralité d'entretoises intérieures distinctes est espacée d'une articulation la plus proche d'environ au moins 25 % de la longueur nette de cette entretoise et la seconde extrémité de chacune parmi ladite pluralité d'entretoises intérieures distinctes est espacée d'une articulation la plus proche d'environ au moins 25 % de la longueur nette de cette entretoise.

2. Structure de support articulée biocompatible selon la revendication 1, dans laquelle la première extrémité de chacune parmi la pluralité d'entretoises extérieures distinctes (1804) est espacée d'une articulation la plus proche d'environ au moins 25 % de sa longueur nette.

3. Structure de support articulée biocompatible selon la revendication 2, dans laquelle la seconde extrémité de chacune parmi la pluralité d'entretoises extérieures distinctes (1804) est espacée d'une articulation la plus proche d'environ au moins 25 % de sa longueur nette.
